# EUROPEAN PATENT SPECIFICATION

(11) **EP 2 802 662 B1**
(45) Date of publication and mention of the grant of the patent: **26.12.2018**
(21) Application number: 13700169.9
(22) Date of filing: 10.01.2013
(51) Int. Cl.: C12N 15/867, C07K 14/12, C07K 16/24, C07K 16/28, C12N 5/0735, C12N 5/0797, C07K 7/16, C12N 5/00

(54) **USE OF PSEUDOTYPED LENTIVIRAL PARTICLES FOR IN VITRO TARGETED TRANSDUCTION OF UNDIFFERENTIATED PLURIPOTENT HUMAN EMBRYONIC STEM CELLS AND INDUCED PLURIPOTENT STEM CELLS**
VERWENDUNG PSEUDOTYPISIERTER LENTIVIRUSPARTIKEL ZUR ZIELGERICHTETEN IN-VITRO-TRANSDUKTION VON UNDIFFERENZIERTEN PLURIPOTENTEN MENSCHLICHEN EMBRYOSTAMMZELLEN UND INDUZIERTEN PLURIPOTENTEN STAMMZELLEN
UTILISATION DE PARTICULES DE LENTIVIRUS PSEUDOTYPÉES POUR LA TRANSDUCTION CIBLÉE IN VITRO DE CELLULES SOUCHES EMBRYONNAIRES HUMAINES PLURIPOTENTES INDIFFÉRENCIÉES ET CELLULES SOUCHES PLURIPOTENTES INDUITES

(30) Priority: 11.01.2012 EP 12000142
(43) Date of publication of application: 19.11.2014
(73) Proprietor: Paul-Ehrlich-Institut Bundesamt für Sera und Impfstoffe, 63225 Langen (DE)
(72) Inventor: SCHNEIDER, Irene, 63456 Hanau (DE); BUCHHOLZ, Christian, 60594 Frankfurt (DE); SCHUMANN, Gerald, 63225 Langen (DE); KLAWITTER, Sabine, 67433 Neustadt/Weinstrasse (DE)
(74) Representative: Grund, Martin
(86) International application number: PCT/EP2013/050426
(87) International publication number: WO 2013/104728

(56) References cited:
- WO-A1-03/056019
- WO-A2-2008/037458
- S FUNKE ET AL: "Pseudotyping lentiviral vectors with the wild-type measles virus glycoproteins improves titer and selectivity", GENE THERAPY, vol. 16, no. 5, 1 May 2009 (2009-05-01), pages 700-705, XP055033658, ISSN: 0969-7128, DOI: 10.1038/gt.2009.11
- FUNKE SABRINA ET AL: "HIV-1 Derived Vectors Pseudotyped with Measles Virus Glycoproteins: Targeting and Transduction of Resting Cells", MOLECULAR THERAPY, vol. 17, no. Suppl. 1, May 2009 (2009-05), pages S1-S2, XP008153816, & 12TH ANNUAL MEETING OF THE AMERICAN SOCIETY OF GENE THERAPY; SAN DIEGO, CA, USA; MAY 27 -30, 2009 ISSN: 1525-0016
- BRIGITTE ANLIKER ET AL: "Specific gene transfer to neurons, endothelial cells and hematopoietic progenitors with lentiviral vectors", NATURE METHODS, vol. 7, no. 11, 1 November 2010 (2010-11-01), pages 929-935, XP055033622, ISSN: 1548-7091, DOI: 10.1038/nmeth.1514
- JANG JUNG-EUN ET AL: "Specific and stable gene transfer to human embryonic stem cells using pseudotyped lentiviral vectors.", STEM CELLS AND DEVELOPMENT FEB 2006 LNKD- PUBMED:16522168, vol. 15, no. 1, February 2006 (2006-02), pages 109-117, XP008153783, ISSN: 1547-3287
- SUZANNE KADEREIT ET AL: "In vitro immunogenicity of undifferentiated pluripotent stem cells (PSC) and derived lineages", SEMINARS IN IMMUNOPATHOLOGY, SPRINGER, BERLIN, DE, vol. 33, no. 6, 5 April 2011 (2011-04-05), pages 551-562, XP019969947, ISSN: 1863-2300, DOI: 10.1007/S00281-011-0265-9
- MATEIZEL I ET AL: "Characterization of CD30 expression in human embryonic stem cell lines cultured in serum-free media and passaged mechanically", HUMAN REPRODUCTION (OXFORD), vol. 24, no. 10, October 2009 (2009-10), pages 2477-2489 URL, XP008153800,
- LAGARKOVA MARIA A ET AL: "CD 30 is a marker of undifferentiated human embryonic stem cells rather than a biomarker of transformed hESCs", CELL CYCLE, LANDES BIOSCIENCE, US, vol. 7, no. 22, 15 November 2008 (2008-11-15), pages 3610-3612, XP009152240, ISSN: 1551-4005, DOI: 10.4161/CC.7.22.6981
- T.-Y. LU ET AL: "Epithelial Cell Adhesion Molecule Regulation Is Associated with the Maintenance of the Undifferentiated Phenotype of Human Embryonic Stem Cells", JOURNAL OF BIOLOGICAL CHEMISTRY, vol. 285, no. 12, 19 March 2010 (2010-03-19), pages 8719-8732, XP055033615, ISSN: 0021-9258, DOI: 10.1074/jbc.M109.077081
- C. GRANDELA ET AL: "hESC Adaptation, Selection and Stability", STEM CELL REVIEWS AND REPORTS, vol. 3, no. 3, 20 September 2007 (2007-09-20), pages 183-191, XP055033613, ISSN: 1550-8943, DOI: 10.1007/s12015-007-0008-4
- DANIELLA HERSZFELD ET AL: "CD30 is a survival factor and a biomarker for transformed human pluripotent stem cells", NATURE BIOTECHNOLOGY, vol. 24, no. 3, 1 March 2006 (2006-03-01), pages 351-357, XP055033614, ISSN: 1087-0156, DOI: 10.1038/nbt1197
- BUCHHOLZ C J ET AL: "Lentiviral vectors with measles virus glycoproteins - dream team for gene transfer?", TRENDS IN BIOTECHNOLOGY, ELSEVIER PUBLICATIONS, CAMBRIDGE, GB, vol. 27, no. 5, 1 May 2009 (2009-05-01), pages 259-265, XP026018665, ISSN: 0167-7799, DOI: 10.1016/J.TIBTECH.2009.02.002 [retrieved on 2009-03-25]
- Anonymous: "Protocol for Transduction of Human Embryonic Stem Cells (hESCs) using Lentiviral Vectors | Sigma-Aldrich", , 3 November 2008 (2008-11-03), XP55234853, Retrieved from the Internet: URL:http://www.sigmaaldrich.com/technical- documents/articles/biofiles/protocol-for-t ransduction.html [retrieved on 2015-12-09]
- Gropp, M. & Reubinoff, B.: "Lentiviral vector-mediated gene delivery into human embryonic stem cells" In: Klimanskaya, I; Lanza, R: "Methods in Enzymology", 2006, METHODS IN ENZYMOLOGY ELSEVIER ACADEMIC PRESS INC, 525 B STREET, SUITE 1900, SAN DIEGO, CA 92101-4495 USA SERIES : METHODS IN ENZYMOLOGY (ISSN 0076-6879(PRINT)), PAGE(S) 64-81, XP008178403, ISSN: null ISBN: 0-12-373651-X pages 64-81, * abstract * * page 67 - page 80; figures 1, 2 *
- THORSTEN FRIEDEL ET AL: "CD30 Receptor-Targeted Lentiviral Vectors for Human Induced Pluripotent Stem Cell-Specific Gene Modification", STEM CELLS AND DEVELOPMENT, vol. 25, no. 9, 1 May 2016 (2016-05-01), pages 729-739, XP055354725, NL ISSN: 1547-3287, DOI: 10.1089/scd.2015.0386
- Dai-Tze Wu ET AL: "Antibody-Directed Lentiviral Gene Transduction for Live-Cell Monitoring and Selection of Human iPS and hES Cells", PLoS ONE, vol. 7, no. 4, 20 April 2012 (2012-04-20), page e34778, XP055355097, DOI: 10.1371/journal.pone.0034778

### SEQUENCE LISTING

<110> Paul-Ehrlich-Institut Bundesamt für Sera und Impfstoffe
   SCHNEIDER, Irene
   BUCHHOLZ, Christian
   SCHUMANN, Gerald
   KLAWITTER, Sabine
<120> Novel pseudotyped lentiviral particles and their use in the in vitro targeted transduction of undifferentiated pluripotent human embryonic stem cells and induced pluripotent stem cells
<130> 141-008P
<150> EP 12000142.5
   <151> 2012-01-11
<160> 17
<170> BiSSAP 1.0
<210> 1
   <211> 246
   <212> PRT
   <213> null
<220>
   <221> SOURCE
   <222> 1..246
   <223> /mol_type="protein"
   /note="scFV-antiCD30"
   /organism=null
<400> 1
   Gln Val Gln Leu Gln Gln Ser Gly Ala Glu Leu Ala Arg Pro Gly Ala
   1               5                   10                  15
   Ser Val Lys Met Ser Cys Lys Ala Ser Gly Tyr Thr Phe Thr Thr Tyr
               20                  25                  30
   Thr Ile His Trp Val Arg Gln Arg Pro Gly His Asp Leu Glu Trp Ile
           35                  40                  45
   Gly Tyr Ile Asn Pro Ser Ser Gly Tyr Ser Asp Tyr Asn Gln Asn Phe
       50                  55                  60
   Lys Gly Lys Thr Thr Leu Thr Ala Asp Lys Ser Ser Asn Thr Ala Tyr
   65                  70                  75                  80
   Met Gln Leu Asn Ser Leu Thr Ser Glu Asp Ser Ala Val Tyr Tyr Cys
                   85                  90                  95
   Ala Arg Arg Ala Asp Tyr Gly Asn Tyr Glu Tyr Thr Trp Phe Ala Tyr
               100                 105                 110
   Trp Gly Gln Gly Thr Thr Val Thr Val Ser Ser Gly Gly Gly Gly Ser
           115                 120                 125
   Gly Gly Gly Gly Ser Gly Gly Gly Gly Ser Asp Ile Glu Leu Thr Gln
       130                 135                 140
   Ser Pro Lys Phe Met Ser Thr Ser Val Gly Asp Arg Val Asn Val Thr
   145                 150                 155                 160
   Tyr Lys Ala Ser Gln Asn Val Gly Thr Asn Val Ala Trp Phe Gln Gln
                   165                 170                 175
   Lys Pro Gly Gln Ser Pro Lys Val Leu Ile Tyr Ser Ala Ser Tyr Arg
               180                 185                 190
   Tyr Ser Gly Val Pro Asp Arg Phe Thr Gly Ser Gly Ser Gly Thr Asp
                   195                 200                 205
   Phe Thr Leu Thr Ile Ser Asn Val Gln Ser Glu Asp Leu Ala Glu Tyr
       210                 215                 220
   Phe Cys Gln Gln Tyr His Thr Tyr Pro Leu Thr Phe Gly Gly Gly Thr
   225                 230                 235                 240
   Lys Leu Glu Ile Lys Arg
                   245
<210> 2
   <211> 246
   <212> PRT
   <213> null
<220>
   <221> SOURCE
   <222> 1..246
   <223> /mol_type="protein"
   /note="corrected scFV-antiCD30opt"
   /organism=null
<400> 2
   Gln Val Gln Leu Val Gln Ser Gly Ala Glu Asp Val Arg Pro Gly Ala
   1               5                   10                  15
   Ser Val Lys Leu Ser Cys Lys Ala Ser Gly Tyr Thr Phe Thr Thr Tyr
               20                  25                  30
   Thr Ile His Trp Val Arg Gln Arg Pro Gly Gln Gly Leu Glu Trp Ile
           35                  40                  45
   Gly Tyr Ile Asn Pro Ser Ser Gly Tyr Ser Asp Tyr Asn Gln Lys Phe
       50                  55                  60
   Lys Gly Lys Thr Thr Leu Thr Ala Asp Lys Ser Ser Asn Thr Ala Tyr
   65                  70                  75                  80
   Met Gln Leu Asn Ser Leu Thr Ser Glu Asp Ser Ala Val Tyr Tyr Cys
                   85                  90                  95
   Ala Arg Arg Ala Asp Tyr Gly Asn Tyr Glu Tyr Thr Trp Phe Ala Tyr
               100                 105                 110
   Trp Gly Gln Gly Thr Thr Val Thr Val Ser Ser Gly Gly Gly Gly Ser
           115                 120                 125
   Gly Gly Gly Gly Ser Gly Gly Gly Gly Ser Asp Ile Val Met Thr Gln
       130                 135                 140
   Ser Pro Lys Ser Met Ser Thr Ser Val Gly Asp Arg Val Thr Ile Thr
   145                 150                 155                 160
   Cys Lys Ala Ser Gln Asn Val Gly Thr Asn Val Ala Trp Tyr Gln Gln
                   165                 170                 175
   Lys Pro Gly Gln Ser Pro Lys Val Leu Ile Tyr Ser Ala Ser Tyr Arg
               180                 185                 190
   Tyr Ser Gly Val Pro Asp Arg Phe Thr Gly Ser Gly Ser Gly Thr Asp
           195                 200                 205
   Phe Thr Leu Thr Ile Ser Asn Val Gln Pro Glu Asp Leu Ala Thr Tyr
       210                 215                 220
   Tyr Cys Gln Gln Tyr His Thr Tyr Pro Leu Thr Phe Gly Gly Gly Thr
   225                 230                 235                 240
   Lys Leu Glu Ile Lys Arg
                   245
<210> 3
   <211> 744
   <212> DNA
   <213> null
<220>
   <221> source
   <222> 1..744
   <223> /mol_type="DNA" /note="corrected scFV-antiCD30opt" /organism=null
<400> 3
   atggcccagg tgcaactggt gcagtcaggg gctgatgtgg ctagacctgg tgcttcagtg      60
   aagttgtcct gcaaggcttc tggctacacc tttactacct acacaataca ctgggtaaga     120
   cagaggcctg gacagggcct ggaatggatt ggatacatta atcctagcag tggatattct     180
   gactacaatc agaaattcaa gggcaagacc acattgactg cagacaagtc ctccaacaca     240
   gcctacatgc aactgaacag cctgacatct gaggactctg cggtctatta ctgtgcaaga     300
   agagcggact atggtaacta cgaatatacc tggtttgctt actggggcca agggaccacg     360
   gtcaccgtct cctcaagtgg aggcggttca ggtggaggtg gctctggcgg tggcggatcg     420
   gacatcgtga tgactcagtc tccaaaatcc atgtcgacat cagtaggaga cagggtcacc     480
   attacctgca aggccagtca gaatgtgggt actaatgtag cctggtatca acagaaaccc     540
   gggcagtctc ctaaacttct gatttactcg gcatcttacc gatacagtgg agtccctgat     600
   cgcttcacag gcagtggatc tggaacagat ttcactctca ccatcagcaa tgtgcagccc     660
   gaggacctgg caacatatta ctgtcaacaa tatcacacct atcctctcac gttcggaggg     720
   ggcaccaagc tggaaatcaa acgg                                            744
<210> 4
   <211> 1797
   <212> DNA
   <213> null
<220>
   <221> source
   <222> 1..1797
   <223> /mol_type="DNA"
   /note="Hc-delta-18"
   /organism=null
<400> 4
   atgggaagta ggatagtcat taacagagaa catcttatga ttgatagacc ttatgttttg      60
   ctggctgttc tgtttgtcat gtttctgagc ttgatcgggt tgctagccat tgcaggaatt     120
   cgacttcatc gggcagccat ctacaccgca gagatccata aaagcctcag caccaatcta     180
   gatgtaacta actcaatcga gcatcaggtc aaggacgtgc tgacaccact cttcaaaatc     240
   atcggtgatg aagtgggcct gaggacacct cagagattca ctgacctagt gaaattcatc     300
   tctgacaaga ttaaattcct taatccggat agggagtacg acttcagaga tctcacttgg     360
   tgtatcaacc cgccagagag aatcaaattg gattatgatc aatactgtgc agatgtggct     420
   gctgaagagc tcatgaatgc attggtgaac tcaactctac tggagaccag aacaaccaat     480
   cagttcctag ctgtctcaaa gggaaactgc tcagggccca ctacaatcag aggtcaattc     540
   tcaaacatgt cgctgtccct gttagacttg tatttaggtc gaggttacaa tgtgtcatct     600
   atagtcacta tgacatccca gggaatgtat gggggaactt acctagtgga aaagcctaat     660
   ctgagcagca aaaggtcaga gttgtcacaa ctgagcatgt accgagtgtt tgaagtaggt     720
   gttatcagaa atccgggttt gggggctccg gtgttccata tgacaaacta tcttgagcaa     780
   ccagtcagta atgatctcag caactgtatg gtggctttgg gggagctcaa actcgcagcc     840
   ctttgtcacg gggaagattc tatcacaatt ccctatcagg gatcagggaa aggtgtcagc     900
   ttccagctcg tcaagctagg tgtctggaaa tccccaaccg acatgcaatc ctgggtcccc     960
   ttatcaacgg atgatccagt gatagacagg ctttacctct catctcacag aggtgttatc    1020
   gctgacaacc aagcaaaatg ggctgtcccg acaacacgaa cagatgacaa gttgcgaatg    1080
   gagacatgct tccaacaggc gtgtaagggt aaaatccaag cactctgcga gaatcccgag    1140
   tgggcaccat tgaaggataa caggattcct tcatacgggg tcttgtctgt tgatctgagt    1200
   ctgacagttg agcttaaaat caaaattgct tcgggattcg ggccattgat cacacacggt    1260
   tcagggatgg acctatacaa atccaaccac aacaatgtgt attggctgac tatcccgcca    1320
   atgaagaacc tagccttagg tgtaatcaac acattggagt ggataccgag attcaaggtt    1380
   agtccctacc tcttcactgt cccaattaag gaagcaggcg gagactgcca tgccccaaca    1440
   tacctacctg cggaggtgga tggtgatgtc aaactcagtt ccaatctggt gattctacct    1500
   ggtcaagatc tccaatatgt tttggcaacc tacgatactt ccagggttga acatgctgtg    1560
   gtttattacg tttacagccc aagccgctca ttttcttact tttatccttt taggttgcct    1620
   ataaaggggg tccccatcga attacaagtg gaatgcttca catgggacca aaaactctgg    1680
   tgccgtcact tctgtgtgct tgcggactca gaatctggtg gacatatcac tcactctggg    1740
   atggtgggca tgggagtcag ctgcacagtc acccgggaag atggaaccaa tcgcaga       1797
<210> 5
   <211> 1793
   <212> DNA
   <213> null
<220>
   <221> source
   <222> 1..1793
   <223> /mol_type="DNA"
   /note="Hc-delta-19"
   /organism=null
<400> 5
   atgagtagga tagtcattaa cagagaacat cttatgattg atagacctta tgttttgctg      60
   gctgttctgt ttgtcatgtt tctgagcttg atcgggttgc tagccattgc aggaattcga     120
   cttcatcggg cagccatcta caccgcagag atccataaaa gcctcagcac caatctagat     180
   gtaactaact caatcgagca tcaggtcaag gacgtgctga caccactctt caaaatcatc     240
   ggtgatgaag tgggcctgag gacacctcag agattcactg acctagtgaa attcatctct 300
   gacaagatta aattccttaa tccggatagg gagtacgact tcagagatct cacttggtgt 360
   atcaacccgc cagagagaat caaattggat tatgatcaat actgtgcaga tgtggctgct 420
   gaagagctca tgaatgcatt ggtgaactca actctactgg agaccagaac aaccaatcag 480
   ttcctagctg tctcaaaggg aaactgctca gggcccacta caatcagagg tcaattctca 540
   aacatgtcgc tgtccctgtt agacttgtat ttaggtcgag gttacaatgt gtcatctata 600
   gtcactatga catcccaggg aatgtatggg ggaacttacc tagtggaaaa gcctaatctg 660
   agcagcaaaa ggtcagagtt gtcacaactg agcatgtacc gagtgtttga agtaggtgtt 720
   atcagaaatc cgggtttggg ggctccggtg ttccatatga caaactactt gagcaaccag 780
   tcagtaatga tctcagcaac tgtatggtgg ctttggggga gctcaaactc gcagcccttt 840
   gtcacgggga agattctatc acaattccct atcagggatc agggaaaggt gtcagcttcc 900
   agctcgtcaa gctaggtgtc tggaaatccc caaccgacat gcaatcctgg gtccccttat 960
   caacggatga tccagtgata gacaggcttt acctctcatc tcacagaggt gttatcgctg 1020
   acaaccaagc aaaatgggct gtcccgacaa cacgaacaga tgacaagttg cgaatggaga 1080
   catgcttcca acaggcgtgt aagggtaaaa tccaagcact ctgcgagaat cccgagtggg 1140
   caccattgaa ggataacagg attccttcat acggggtctt gtctgttgat ctgagtctga 1200
   cagttgagct taaaatcaaa attgcttcgg gattcgggcc attgatcaca cacggttcag 1260
   ggatggacct atacaaatcc aaccacaaca atgtgtattg gctgactatc ccgccaatga 1320
   agaacctagc cttaggtgta atcaacacat tggagtggat accgagattc aaggttagtc 1380
   cctacctctt cactgtccca attaaggaag caggcggaga ctgccatgcc ccaacatacc 1440
   tacctgcgga ggtggatggt gatgtcaaac tcagttccaa tctggtgatt ctacctggtc 1500
   aagatctcca atatgttttg gcaacctacg atacttccag ggttgaacat gctgtggttt 1560
   attacgttta cagcccaagc cgctcatttt cttactttta tccttttagg ttgcctataa 1620
   agggggtccc catcgaatta caagtggaat gcttcacatg ggaccaaaaa ctctggtgcc 1680
   gtcacttctg tgtgcttgcg gactcagaat ctggtggaca tatcactcac tctgggatgg 1740
   tgggcatggg agtcagctgc acagtcaccc gggaagatgg aaccaatcgc aga 1793
<210> 6
   <211> 1791
   <212> DNA
   <213> null
<220>
   <221> source
   <222> 1..1791
   <223> /mol_type="DNA"
   /note="Hc-delta-24+4A"
   /organism=null
<400> 6
   atggctgccg cagcgaacag agaacatctt atgattgata gaccttatgt tttgctggct      60
   gttctgtttg tcatgtttct gagcttgatc gggttgctag ccattgcagg aattcgactt     120
   catcgggcag ccatctacac cgcagagatc cataaaagcc tcagcaccaa tctagatgta     180
   actaactcaa tcgagcatca ggtcaaggac gtgctgacac cactcttcaa aatcatcggt     240
   gatgaagtgg gcctgaggac acctcagaga ttcactgacc tagtgaaatt catctctgac     300
   aagattaaat tccttaatcc ggatagggag tacgacttca gagatctcac ttggtgtatc     360
   aacccgccag agagaatcaa attggattat gatcaatact gtgcagatgt ggctgctgaa     420
   gagctcatga atgcattggt gaactcaact ctactggaga ccagaacaac caatcagttc     480
   ctagctgtct caaagggaaa ctgctcaggg cccactacaa tcagaggtca attctcaaac     540
   atgtcgctgt ccctgttaga cttgtattta ggtcgaggtt acaatgtgtc atctatagtc     600
   actatgacat cccagggaat gtatggggga acttacctag tggaaaagcc taatctgagc     660
   agcaaaaggt cagagttgtc acaactgagc atgtaccgag tgtttgaagt aggtgttatc     720
   agaaatccgg gtttgggggc tccggtgttc catatgacaa actatcttga gcaaccagtc     780
   agtaatgatc tcagcaactg tatggtggct ttgggggagc tcaaactcgc agccctttgt     840
   cacggggaag attctatcac aattccctat cagggatcag ggaaaggtgt cagcttccag     900
   ctcgtcaagc taggtgtctg gaaatcccca accgacatgc aatcctgggt ccccttatca     960
   acggatgatc cagtgataga caggctttac ctctcatctc acagaggtgt tatcgctgac    1020
   aaccaagcaa aatgggctgt cccgacaaca cgaacagatg acaagttgcg aatggagaca    1080
   tgcttccaac aggcgtgtaa gggtaaaatc caagcactct gcgagaatcc cgagtgggca    1140
   ccattgaagg ataacaggat tccttcatac ggggtcttgt ctgttgatct gagtctgaca    1200
   gttgagctta aaatcaaaat tgcttcggga ttcgggccat tgatcacaca cggttcaggg    1260
   atggacctat acaaatccaa ccacaacaat gtgtattggc tgactatccc gccaatgaag    1320
   aacctagcct taggtgtaat caacacattg gagtggatac cgagattcaa ggttagtccc    1380
   tacctcttca ctgtcccaat taaggaagca ggcggagact gccatgcccc aacataccta    1440
   cctgcggagg tggatggtga tgtcaaactc agttccaatc tggtgattct acctggtcaa    1500
   gatctccaat atgttttggc aacctacgat acttccaggg ttgaacatgc tgtggtttat    1560
   tacgtttaca gcccaagccg ctcattttct tacttttatc cttttaggtt gcctataaag    1620
   ggggtcccca tcgaattaca agtggaatgc ttcacatggg accaaaaact ctggtgccgt    1680
   cacttctgtg tgcttgcgga ctcagaatct ggtggacata tcactcactc tgggatggtg    1740
   ggcatgggag tcagctgcac agtcacccgg gaagatggaa ccaatcgcag a             1791
<210> 7
   <211> 599
   <212> PRT
   <213> null
<220>
   <221> SOURCE
   <222> 1..599
   <223> /mol_type="protein"
   /note="Hc-delta-18"
   /organism=null
<400> 7
   Met Gly Ser Arg Ile Val Ile Asn Arg Glu His Leu Met Ile Asp Arg
   1               5                   10                  15
   Pro Tyr Val Leu Leu Ala Val Leu Phe Val Met Phe Leu Ser Leu Ile
               20                  25                  30
   Gly Leu Leu Ala Ile Ala Gly Ile Arg Leu His Arg Ala Ala Ile Tyr
           35                  40                  45
   Thr Ala Glu Ile His Lys Ser Leu Ser Thr Asn Leu Asp Val Thr Asn
       50                  55                  60
   Ser Ile Glu His Gln Val Lys Asp Val Leu Thr Pro Leu Phe Lys Ile
   65                  70                  75                  80
   Ile Gly Asp Glu Val Gly Leu Arg Thr Pro Gln Arg Phe Thr Asp Leu
                   85                  90                  95
   Val Lys Phe Ile Ser Asp Lys Ile Lys Phe Leu Asn Pro Asp Arg Glu
               100                 105                 110
   Tyr Asp Phe Arg Asp Leu Thr Trp Cys Ile Asn Pro Pro Glu Arg Ile
           115                 120                 125
   Lys Leu Asp Tyr Asp Gln Tyr Cys Ala Asp Val Ala Ala Glu Glu Leu
       130                 135                 140
   Met Asn Ala Leu Val Asn Ser Thr Leu Leu Glu Thr Arg Thr Thr Asn
   145                 150                 155                 160
   Gln Phe Leu Ala Val Ser Lys Gly Asn Cys Ser Gly Pro Thr Thr Ile
                   165                 170                 175
   Arg Gly Gln Phe Ser Asn Met Ser Leu Ser Leu Leu Asp Leu Tyr Leu
               180                 185                 190
   Gly Arg Gly Tyr Asn Val Ser Ser Ile Val Thr Met Thr Ser Gln Gly
           195                 200                 205
   Met Tyr Gly Gly Thr Tyr Leu Val Glu Lys Pro Asn Leu Ser Ser Lys
       210                 215                 220
   Arg Ser Glu Leu Ser Gln Leu Ser Met Tyr Arg Val Phe Glu Val Gly
   225                 230                 235                 240
   Val Ile Arg Asn Pro Gly Leu Gly Ala Pro Val Phe His Met Thr Asn
                   245                 250                 255
   Tyr Leu Glu Gln Pro Val Ser Asn Asp Leu Ser Asn Cys Met Val Ala
               260                 265                 270
   Leu Gly Glu Leu Lys Leu Ala Ala Leu Cys His Gly Glu Asp Ser Ile
           275                 280                 285
   Thr Ile Pro Tyr Gln Gly Ser Gly Lys Gly Val Ser Phe Gln Leu Val
       290                 295                 300
   Lys Leu Gly Val Trp Lys Ser Pro Thr Asp Met Gln Ser Trp Val Pro
   305                 310                 315                 320
   Leu Ser Thr Asp Asp Pro Val Ile Asp Arg Leu Tyr Leu Ser Ser His
                   325                 330                 335
   Arg Gly Val Ile Ala Asp Asn Gln Ala Lys Trp Ala Val Pro Thr Thr
               340                 345                 350
   Arg Thr Asp Asp Lys Leu Arg Met Glu Thr Cys Phe Gln Gln Ala Cys
           355                 360                 365
   Lys Gly Lys Ile Gln Ala Leu Cys Glu Asn Pro Glu Trp Ala Pro Leu
       370                 375                 380
   Lys Asp Asn Arg Ile Pro Ser Tyr Gly Val Leu Ser Val Asp Leu Ser
   385                 390                 395                 400
   Leu Thr Val Glu Leu Lys Ile Lys Ile Ala Ser Gly Phe Gly Pro Leu
                   405                 410                 415
   Ile Thr His Gly Ser Gly Met Asp Leu Tyr Lys Ser Asn His Asn Asn
               420                 425                 430
   Val Tyr Trp Leu Thr Ile Pro Pro Met Lys Asn Leu Ala Leu Gly Val
           435                 440                 445
   Ile Asn Thr Leu Glu Trp Ile Pro Arg Phe Lys Val Ser Pro Tyr Leu
       450                 455                 460
   Phe Thr Val Pro Ile Lys Glu Ala Gly Gly Asp Cys His Ala Pro Thr
   465                 470                 475                 480
   Tyr Leu Pro Ala Glu Val Asp Gly Asp Val Lys Leu Ser Ser Asn Leu
                   485                 490                 495
   Val Ile Leu Pro Gly Gln Asp Leu Gln Tyr Val Leu Ala Thr Tyr Asp
               500                 505                 510
   Thr Ser Arg Val Glu His Ala Val Val Tyr Tyr Val Tyr Ser Pro Ser
           515                 520                 525
   Arg Ser Phe Ser Tyr Phe Tyr Pro Phe Arg Leu Pro Ile Lys Gly Val
       530                 535                 540
   Pro Ile Glu Leu Gln Val Glu Cys Phe Thr Trp Asp Gln Lys Leu Trp
   545                 550                 555                 560
   Cys Arg His Phe Cys Val Leu Ala Asp Ser Glu Ser Gly Gly His Ile
                   565                 570                 575
   Thr His Ser Gly Met Val Gly Met Gly Val Ser Cys Thr Val Thr Arg
               580                 585                 590
   Glu Asp Gly Thr Asn Arg Arg
           595
<210> 8
   <211> 1572
   <212> DNA
   <213> null
<220>
   <221> source
   <222> 1..1572
   <223> /mol_type="DNA"
   /note="Fc-delta-30"
   /organism=null
<400> 8
   atgtccatca tgggtctcaa ggtgaacgtc tctgccatat tcatggcagt actgttaact      60
   ctccaaacac ccaccggtca aatccattgg ggcaatctct ctaagatagg ggtggtagga     120
   ataggaagtg caagctacaa agttatgact cgttccagcc atcaatcatt agtcataaaa     180
   ttaatgccca atataactct cctcaataac tgcacgaggg tagagattgc agaatacagg     240
   agactactga gaactgtttt ggaaccaatt agagatgcac ttaatgcaat gacccagaat     300
   ataagaccgg ttcagagtgt agcttcaagt aggagacaca agagatttgc gggagtagtc     360
   ctggcaggtg cggccctagg cgttgccaca gctgctcaga taacagccgg cattgcactt     420
   caccagtcca tgctgaactc tcaagccatc gacaatctga gagcgagcct ggaaactact     480
   aatcaggcaa ttgaggcaat cagacaagca gggcaggaga tgatattggc tgttcagggt     540
   gtccaagact acatcaataa tgagctgata ccgtctatga accaactatc ttgtgattta     600
   atcggccaga agctcgggct caaattgctc agatactata cagaaatcct gtcattattt     660
   ggccccagct tacgggaccc catatctgcg gagatatcta tccaggcttt gagctatgcg     720
   cttggaggag acatcaataa ggtgttagaa aagctcggat acagtggagg tgatttactg     780
   ggcatcttag agagcagagg aataaaggcc cggataactc acgtcgacac agagtcctac     840
   ttcattgtcc tcagtatagc ctatccgacg ctgtccgaga ttaagggggt gattgtccac     900
   cggctagagg gggtctcgta caacataggc tctcaagagt ggtataccac tgtgcccaag     960
   tatgtcgcaa cccaagggta ccttatctcg aattttgatg agtcatcgtg tactttcatg    1020
   ccagagggaa ctgtgtgcag ccaaaatgcc ttgtacccga tgagtcctct gctccaagaa    1080
   tgcctccggg ggtccactaa gtcctgtgct cgtacactcg tatccgggtc ttttgggaac    1140
   cggttcattt tatcacaagg gaacctaata gccaattgtg catcaatcct ttgcaagtgt    1200
   tacacaacag gaacgatcat taatcaagac cctgacaaga tcctaacata cattgctgcc    1260
   gatcactgcc cggtagtcga ggtgaacggc gtgaccatcc aagtcgggag caggaggtat    1320
   ccagacgctg tgtacttgca cagaattgac ctcggtcctc ccatatcatt ggagaggttg    1380
   gacgtaggga caaatctggg gaatgcaatt gctaagttgg aggatgccaa ggaattgttg    1440
   gagtcatcgg accagatatt gaggagtatg aaaggtttat cgagcactag catagtctac    1500
   atcctgattg cagtgtgtct tggagggttg atagggatcc ccgctttaat atgttgctgc    1560
   agggggcgtt ga                                                        1572
<210> 9
   <211> 522
   <212> PRT
   <213> null
<220>
   <221> SOURCE
   <222> 1..522
   <223> /mol_type="protein"
   /note="Fc-delta-30"
   /organism=null
<400> 9
   Met Ser Ile Met Gly Leu Lys Val Asn Val Ser Ala Ile Phe Met Ala
   1               5                   10                  15
   Val Leu Leu Thr Leu Gln Thr Pro Thr Gly Gln Ile His Trp Gly Asn
               20                  25                  30
   Leu Ser Lys Ile Gly Trp Gly Ile Gly Ser Ala Ser Tyr Lys Val Met
           35                  40                  45
   Thr Arg Ser Ser His Gln Ser Leu Val Ile Lys Leu Met Pro Asn Ile
       50                  55                  60
   Thr Leu Leu Asn Asn Cys Thr Arg Val Glu Ile Ala Glu Tyr Arg Arg
   65                  70                  75                  80
   Leu Leu Arg Thr Val Leu Glu Pro Ile Arg Asp Ala Leu Asn Ala Met
                   85                  90                  95
   Thr Gln Asn Ile Arg Pro Val Gln Ser Val Ala Ser Ser Arg Arg His
               100                 105                 110
   Lys Arg Phe Ala Gly Val Val Leu Ala Gly Ala Ala Leu Gly Val Ala
           115                 120                 125
   Thr Ala Ala Gln Ile Thr Ala Gly Ile Ala Leu His Gln Ser Met Leu
           130                 135                 140
   Asn Ser Gln Ala Ile Asp Asn Leu Arg Ala Ser Leu Glu Thr Thr Asn
   145                 150                 155                 160
   Gln Ala Ile Glu Ala Ile Arg Gln Ala Gly Gln Glu Met Ile Leu Ala
                   165                 170                 175
   Val Gln Gly Val Gln Asp Tyr Ile Asn Asn Glu Leu Ile Pro Ser Met
               180                 185                 190
   Asn Gln Leu Ser Cys Asp Leu Ile Gly Gln Lys Leu Gly Leu Lys Leu
           195                 200                 205
   Leu Arg Tyr Tyr Thr Glu Ile Leu Ser Leu Phe Gly Pro Ser Leu Arg
       210                 215                 220
   Asp Pro Ile Ser Ala Glu Ile Ser Ile Gln Ala Leu Ser Tyr Ala Leu
   225                 230                 235                 240
   Gly Gly Asp Ile Asn Lys Val Leu Glu Lys Leu Gly Tyr Ser Gly Gly
                   245                 250                 255
   Asp Leu Leu Gly Ile Leu Glu Ser Arg Gly Ile Lys Ala Arg Ile Thr
               260                 265                 270
   His Val Asp Thr Glu Ser Tyr Phe Ile Val Leu Ser Ile Ala Tyr Pro
           275                 280                 285
   Thr Leu Ser Glu Ile Lys Gly Val Ile Val His Arg Leu Glu Gly Val
       290                 295                 300
   Ser Tyr Asn Ile Gly Ser Gln Glu Trp Tyr Thr Thr Val Pro Lys Tyr
   305                 310                 315                 320
   Val Ala Thr Gln Gly Tyr Leu Ile Ser Asn Phe Asp Glu Ser Ser Cys
                   325                 330                 335
   Thr Phe Met Pro Glu Gly Thr Val Cys Ser Gln Asn Ala Leu Tyr Pro
               340                 345                 350
   Met Ser Pro Leu Leu Gln Glu Cys Leu Arg Gly Ser Thr Lys Ser Cys
           355                 360                 365
   Ala Arg Thr Leu Val Ser Gly Ser Phe Gly Asn Arg Phe Ile Leu Ser
       370                 375                 380
   Gln Gly Asn Leu Ile Ala Asn Cys Ala Ser Ile Leu Cys Lys Cys Tyr
   385                 390                 395                 400
   Thr Thr Gly Thr Ile Ile Asn Gln Asp Pro Asp Lys Ile Leu Thr Tyr
                   405                 410                 415
   Ile Ala Ala Asp His Cys Pro Val Val Glu Val Asn Gly Val Thr Ile
               420                 425                 430
   Gln Val Gly Ser Arg Arg Tyr Pro Asp Ala Val Tyr Leu His Arg Ile
           435                 440                 445
   Asp Leu Gly Pro Pro Ile Ser Leu Glu Arg Leu Asp Val Gly Thr Asn
       450                 455                 460
   Leu Gly Asn Ala Ile Ala Lys Leu Glu Asp Ala Lys Glu Leu Leu Glu
   465                 470                 475                 480
   Ser Ser Asp Gln Ile Leu Arg Ser Met Lys Gly Leu Ser Ser Thr Ser
                   485                 490                 495
   Ile Val Tyr Ile Leu Ile Ala Val Cys Leu Gly Gly Leu Ile Gly Ile
               500                 505                 510
   Pro Ala Leu Ile Cys Cys Cys Arg Gly Arg
           515                 520
<210> 10
   <211> 1791
   <212> DNA
   <213> null
<220>
   <221> source
   <222> 1..1791
   <223> /mol_type="DNA"
   /note="Hc-delta-24+4A_b"
   /organism=null
<400> 10
   atggctgccg cagcgaacag agaacatctt atgattgata gaccttatgt tttgctggct 60
   gttctgtttg tcatgtttct gagcttgatc gggttgctag ccattgcagg aattcgactt 120
   catcgggcag ccatctacac cgcagagatc cataaaagcc tcagcaccaa tctagatgta 180
   actaactcaa tcgagcatca ggtcaaggac gtgctgacac cactcttcaa aatcatcggt 240
   gatgaagtgg gcctgaggac acctcagaga ttcactgacc tagtgaaatt catctctgac 300
   aagattaaat tccttaatcc ggatagggag tacgacttca gagatctcac ttggtgtatc 360
   aacccgccag agagaatcaa attggattat gatcaatact gtgcagatgt ggctgctgaa 420
   gagctcatga atgcattggt gaactcaact ctactggaga ccagaacaac caatcagttc 480
   ctagctgtct caaagggaaa ctgctcaggg cccactacaa tcagaggtca attctcaaac 540
   atgtcgctgt ccctgttaga cttgtattta ggtcgaggtt acaatgtgtc atctatagtc 600
   actatgacat cccagggaat gtatggggga acttacctag tggaaaagcc taatctgagc 660
   agcaaaaggt cagagttgtc acaactgagc atgtaccgag tgtttgaagt aggtgttatc 720
   agaaatccgg gtttgggggc tccggtgttc catatgacaa actatcttga gcaaccagtc 780
   agtaatgatc tcagcaactg tatggtggct ttgggggagc tcaaactcgc agccctttgt 840
   cacggggaag attctatcac aattccctat cagggatcag ggaaaggtgt cagcttccag 900
   ctcgtcaagc taggtgtctg gaaatcccca accgacatgc aatcctgggt ccccttatca 960
   acggatgatc cagtgataga caggctttac ctctcatctc acagaggtgt tatcgctgac 1020
   aaccaagcaa aatgggctgt cccgacaaca cgaacagatg acaagttgcg aatggagaca 1080
   tgcttccaac aggcgtgtaa gggtaaaatc caagcactct gcgagaatcc cgagtgggca 1140
   ccattgaagg ataacaggat tccttcatac ggggtcttgt ctgttgatct gagtctgaca 1200
   gttgagctta aaatcaaaat tgcttcggga ttcgggccat tgatcacaca cggttcaggg 1260
   atggacctat acaaatccaa ccacaacaat gtgtattggc tgactatccc gccaatgaag 1320
   aacctagcct taggtgtaat caacacattg gagtggatac cgagattcaa ggttagtccc 1380
   tacctcttca ctgtcccaat taaggaagca ggcggagact gccatgcccc aacataccta 1440
   cctgcggagg tggatggtga tgtcaaactc agttccaatc tggtgattct acctggtcaa 1500
   gatctccaat atgttttggc aacctacgat acttccaggg ttgaacatgc tgtggtttat 1560
   tacgtttaca gcccaagccg ctcattttct tacttttatc cttttaggtt gcctataaag 1620
   ggggtcccca tcgaattaca agtggaatgc ttcacatggg accaaaaact ctggtgccgt 1680
   cacttctgtg tgcttgcgga ctcagaatct ggtggacata tcactcactc tgggatggtg 1740
   ggcatgggag tcagctgcac agtcacccgg gaagatggaa ccaatcgcag a 1791
<210> 11
   <211> 38
   <212> PRT
   <213> null
<220>
   <221> SOURCE
   <222> 1..38
   <223> /mol_type="protein"
   /note="F protein wildtype"
   /organism=null
<400> 11
   Leu Ile Cys Cys Cys Arg Gly Arg Cys Asn Lys Lys Gly Glu Gln Val
   1               5                   10                  15
   Gly Met Ser Arg Pro Gly Leu Lys Pro Asp Leu Thr Gly Thr Ser Lys
               20                  25                  30
   Ser Tyr Val Arg Ser Leu
           35
<210> 12
   <211> 8
   <212> PRT
   <213> null
<220>
   <221> SOURCE
   <222> 1..8
   <223> /mol_type="protein"
   /note="Fc-delta-30"
   /organism=null
<400> 12
   Leu Ile Cys Cys Cys Arg Gly Arg
   1               5
<210> 13
   <211> 38
   <212> PRT
   <213> null
<220>
   <221> SOURCE
   <222> 1..38
   <223> /mol_type="protein"
   /note="H protein wildtype"
   /organism=null
<400> 13
   Met Ser Pro Gln Arg Asp Arg Ile Asn Ala Phe Tyr Lys Asp Asn Pro
   1               5                   10                  15
   His Pro Lys Gly Ser Arg Ile Val Ile Asn Arg Glu His Leu Met Ile
               20                  25                  30
   Asp Arg Pro Tyr Val Leu
           35
<210> 14
   <211> 20
   <212> PRT
   <213> null
<220>
   <221> SOURCE
   <222> 1..20
   <223> /mol_type="protein" /note="Hc-delta-18" /organism=null
<400> 14
   Met Gly Ser Arg Ile Val Ile Asn Arg Glu His Leu Met Ile Asp Arg
   1               5                   10                  15
   Pro Tyr Val Leu
               20
<210> 15
   <211> 19
   <212> PRT
   <213> null
<220>
   <221> SOURCE
   <222> 1..19
   <223> /mol_type="protein"
   /note="Hc-delta-19"
   /organism=null
<400> 15
   Met Ser Arg Ile Val Ile Asn Arg Glu His Leu Met Ile Asp Arg Pro
   1               5                   10                  15
   Tyr Val Leu
<210> 16
   <211> 18
   <212> PRT
   <213> null
<220>
   <221> SOURCE
   <222> 1..18
   <223> /mol_type="protein"
   /note="Hc-delta-24+4A"
   /organism=null
<400> 16
   Met Ala Ala Ala Ala Asn Arg Glu His Leu Met Ile Asp Arg Pro Tyr
   1               5                   10                  15
   Val Leu
<210> 17
   <211> 248
   <212> PRT
   <213> null
<220>
   <221> SOURCE
   <222> 1..248
   <223> /mol_type="protein"
   /note="CD30opt"
   /organism=null
<400> 17
   Met Ala Gln Val Gln Leu Val Gln Ser Gly Ala Asp Val Ala Arg Pro
   1               5                   10                  15
   Gly Ala Ser Val Lys Leu Ser Cys Lys Ala Ser Gly Tyr Thr Phe Thr
               20                  25                  30
   Thr Tyr Thr Ile His Trp Val Arg Gln Arg Pro Gly Gln Gly Leu Glu
           35                  40                  45
   Trp Ile Gly Tyr Ile Asn Pro Ser Ser Gly Tyr Ser Asp Tyr Asn Gln
       50                  55                  60
   Lys Phe Lys Gly Arg Ala Thr Leu Thr Ala Asp Lys Ser Ser Asn Thr
   65                  70                  75                  80
   Ala Tyr Met Gln Leu Asn Ser Leu Arg Ser Glu Asp Ser Ala Val Tyr
                   85                  90                  95
   Tyr Cys Ala Arg Arg Ala Asp Tyr Gly Asn Tyr Glu Tyr Thr Trp Phe
               100                 105                 110
   Ala Tyr Trp Gly Gln Gly Thr Thr Val Thr Val Ser Ser Ser Gly Gly
           115                 120                 125
   Gly Ser Gly Gly Gly Gly Ser Gly Gly Gly Gly Ser Asp Ile Val Met
       130                 135                 140
   Thr Gln Ser Pro Lys Ser Met Ser Thr Ser Val Gly Asp Arg Val Thr
   145                 150                 155                 160
   Ile Thr Cys Lys Ala Ser Gln Asn Val Gly Thr Asn Val Ala Trp Tyr
                   165                 170                 175
   Gln Gln Lys Pro Gly Gln Ser Pro Lys Leu Leu Ile Tyr Ser Ala Ser
               180                 185                 190
   Tyr Arg Tyr Ser Gly Val Pro Asp Arg Phe Thr Gly Ser Gly Ser Gly
           195                 200                 205
   Thr Asp Phe Thr Leu Thr Ile Ser Asn Val Gln Pro Glu Asp Leu Ala
       210                 215                 220
   Thr Tyr Tyr Cys Gln Gln Tyr His Thr Tyr Pro Leu Thr Phe Gly Gly
   225                 230                 235                 240
   Gly Thr Lys Leu Glu Ile Lys Arg
                   245

## Description

### Technical Field

The present disclosure relates to novel pseudotyped lentiviral particles and to their use in the *in vitro* targeted transduction of undifferentiated pluripotent human embryonic stem cells (hESCs) and induced pluripotent stem cells (iPSCs).

### Prior art

Stem cells are undifferentiated cells found in all multicellular organisms that can divide through mitosis and differentiate into diverse specialized cell types and can self renew to produce more stem cells. They are able to differentiate into a variety of cell types and, accordingly, may be a source of replacement cells and tissues that are damaged in the course of disease, infection, or because of congenital abnormalities (Lovell-Badge, R., Nature, 2001, 414: 88-91). Various types of stem cells exist that are characterized by their differentiation potential *in vivo* and *in vitro,* namely depending on the tissue they are isolated from. They carry out the unique functions of particular tissues when they differentiate in mature cells, wherein pluripotent stem cells are thought to have the potential to differentiate in almost any cell type, while multipotent stem cells are believed to have the potential to differentiate into many cell types only.

Pluripotent stem cells (PSCs) are available for example as undifferentiated embryonic stem cells (ESCs) and can be cultured *in vitro* as permanent lines without undergoing differentiation. They are able to generate all cell types of the body, but not the umbilical cord, trophoblasts and associated structures. Although they are unable to generate a functional organism, they provide the opportunity to develop stem cell-based therapies for human diseases and tissue repair (Leeb, C. et al., Cell Prolif., 2011, 44, Suppl. 1: 9-14). The first successful derivation of human ESCs (hESCs) has been reported in 1998 (Thomson, J. A. et al., Science, 1998, 282: 1145-1147), where the hESCs are derived from the inner cell mass of a blastocyst before it would have implanted in the uterine wall. Gearhart and co-workers derived human embryonic germ (hEGC) cell lines from fetal gonadal tissue (US 6,090,622). Both hESCs and hEGCs have the long-sought characteristics of pluripotent stem cells: they can be cultured extensively without differentiating, they have a normal karyotype, and they are capable of producing a number of important cell types. A significant challenge to the use of pluripotent stem cells for therapy is that they are traditionally cultured on a layer of feeder cells to prevent differentiation (US 5,843,780; US 6,090,622). According to Thomson et al., (supra), hESCs cultures without feeders soon die, or differentiate into a heterogeneous population of committed cells.

Because the aforementioned methods require the destruction of early embryos, alternative sources for hESCs have been exploited for ethical reasons. An alternative pluripotent stem cell type of significance has been introduced in 2006 where *in vitro* cultured somatic cells have been reversed into an embryonic-like pluripotent state leading to the formation of induced pluripotent stem cells (iPSCs; Takahashi, K. & S. Yamanaka, Cell, 2006, 126: 663-676). The reprogramming is performed by introduction of certain stem cell-associated genes, i.e. of 3-5 factors (Krüppel-like factor 4, Klf-4; sex determining region Y-box, Sox, e.g. Sox-2; octamer binding transcription factor 4, Oct4; transcription factor Nanog; transcription factor cMyc) into non-pluripotent, i.e. differentiated cells (e.g. fibroblasts) either by viral transduction or transfection. This reprogramming technology avoids all ethical problems with hESCs and even more important, enables the generation of pluripotent cells for autologous cell therapies as iPSC can be derived directly from the patient for personalised medicine approaches. Furthermore it allows the application of human iPSCs to specify issues in drug research and in safety pharmacological or toxicological studies (Leeb, C. et al., Cell Prolif., 2011, 44, Suppl. 1: 9-14).

So far, many promising studies have shown the therapeutic and scientific potential of stem cells, in particular of hESCs and iPSCs. Progress in hESC and iPSC application was achieved by improving techniques for the gene transfer. Stably transfected stem cells can be used for example as cellular vehicle for protein-supplement gene therapy and/or to direct the stem cells into particular lineages. Furthermore, insertion of lineage-specific reporter constructs would allow isolation of lineage-specific cells, and would allow drug discovery, target validation, and/or stem cell based studies of gene function and the like. However, current strategies of gene transfer do not distinguish between cells of a stem cell phenotype and cells that entered in a differentiation pathway or feeder cells. Additionally, current methods are usually very inefficient and often toxic to the iPSCs, since the cells have to be trypsinised and separated from feeder cells to allow transduction with sufficient efficiency (Jang, J. E. et al., Stem Cells Dev., 2006, 15: 109-17). Only a minor fraction of the cells survives this procedure. A method using adenovirus vector mediated transduction only allows efficient gene transfer when the cells are cultivated in a special medium supplemented with a Rho-kinase inhibitor (Tashiro, K. et al., Cell Reprogramming, 2010, 12: 501-507). Thus, current methodology depends on manipulations of the cells before transduction, which are complex and can reduce the stem cell characteristics of the cells. Gropp, M. and Reubinoff, B. (2006) (Methods in Enzymology, 420, 64 - 81) disclosed lentiviral-based vectors for gene delivery to human embryonic stem cells (hESCs).

### Problem of the invention

Thus, the problem of the present invention is to provide means and/or a method how to achieve targeted gene transfer into undifferentiated human embryonic stem cells and induced pluripotent stem cells, which avoid the disadvantages as mentioned above and are superior with respect to the specificity and efficiency of transduction.

### Summary of the invention

The invention is defined by the appended claims.
The inventors developed novel pseudotyped lentiviral vector particles comprising a morbillivirus fusion (F) protein and a mutated hemagglutinin (H) protein of the measles virus (MeV) or the Edmonton strain of the measles virus (MeV_{Edm}), wherein the cytoplasmic portions of the F and the H protein are truncated, and wherein the amino acids necessary for receptor recognition in the H protein are mutated so that it does not interact with CD46, SLAM and/or nectin-4 and further has a single chain antibody to a cell surface marker of hESCs and iPSCs at its ectodomain. The single chain variable fragment (scFv) anti-cell surface marker coding sequence of the single chain antibody is fused to the coding sequence at the ectodomain of the H protein, wherein the cell surface marker is selected from the group consisting of CD30, EpCAM (CD326), CD9, Thy-1 (CD90), SSEA-3, SSEA-4, TRA-1-60 or TRA-1-81. The transduction according to the present invention does not interfere with the pluripotency, i.e. present transduced hESCs and iPSCs remain undifferentiated, i.e. are able differentiate into all germ layer lineages.

The inventors found out that the desired targeted transduction of hESCs and iPSCs is substantially improved by using lentiviral vector particles pseudotyped with a truncated morbillivirus fusion (F) protein and a mutated and truncated morbillivirus hemagglutinin (H) protein further displaying a single chain antibody to a stem cell surface marker at its ectodomain. In this approach, the single chain antibody is responsible that only those stem cells are transduced which express the respective cell surface marker that is recognized by the antibody. In a preferred embodiment the single-chain variable fragment (scFv) which is fused to the ectodomain of the H protein is an anti-CD30, anti-EpCAM (CD326), anti-CD9, anti-Thy-1 (CD90), anti-SSEA-3, anti-SSEA-4, anti-TRA-1-60, or anti-TRA-1-81 coding sequence, most preferably anti-CD30 coding sequence. These coding sequences are particularly suitable since the expression of the cell surface markers CD30, EpCAM (CD326), CD9, Thy-1 (CD90), SSEA-3, SSEA-4, TRA-1-60, and TRA-1-81 is rapidly down-regulated upon differentiation of hESCs and iPSCs and the transduction does not interfere with the pluripotency, i.e. present transduced hESCs and iPSCs are able differentiate into all germ layer lineages.

Such transfected stem cells can be used for example as cellular vehicle for protein-supplement gene therapy and/or to direct the stem cells into particular lineages. Furthermore, insertion of lineage-specific reporter constructs would allow isolation of lineage-specific cells, and would allow drug discovery, target validation, and/or stem cell based studies of gene function and the like.

### Brief description of the Figures

Figure 1 shows the amino acid sequence of the scFV-antiCD30 (upper sequence lane) and changed amino acids in the corrected scFV-antiCD30ₒₚₜ (lower sequence lane). Critical amino acids in the scFv framework sequence are substituted by the underneath amino acid. Variable complementarity determining regions (CDRs) of the heavy chain variable domain (V_{H}, light gray) and the light chain variable domain (V_{L}, dark gray) and the linker between V_{H} and V_{L} (black) are indicated.

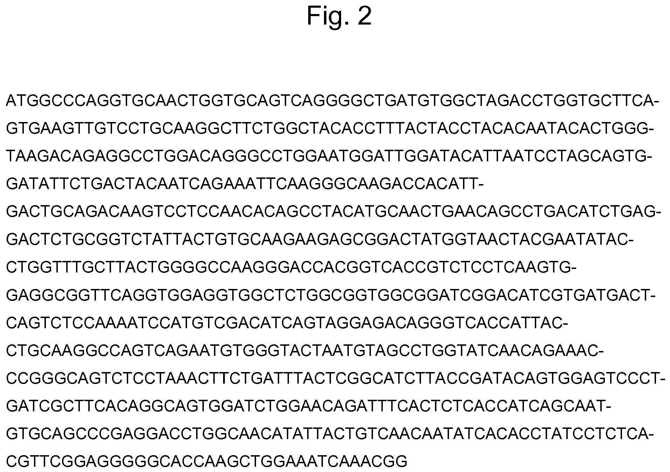

Figure 2 shows the corresponding DNA sequence to the corrected/optimized scFV-antiCD30 of Fig. 1

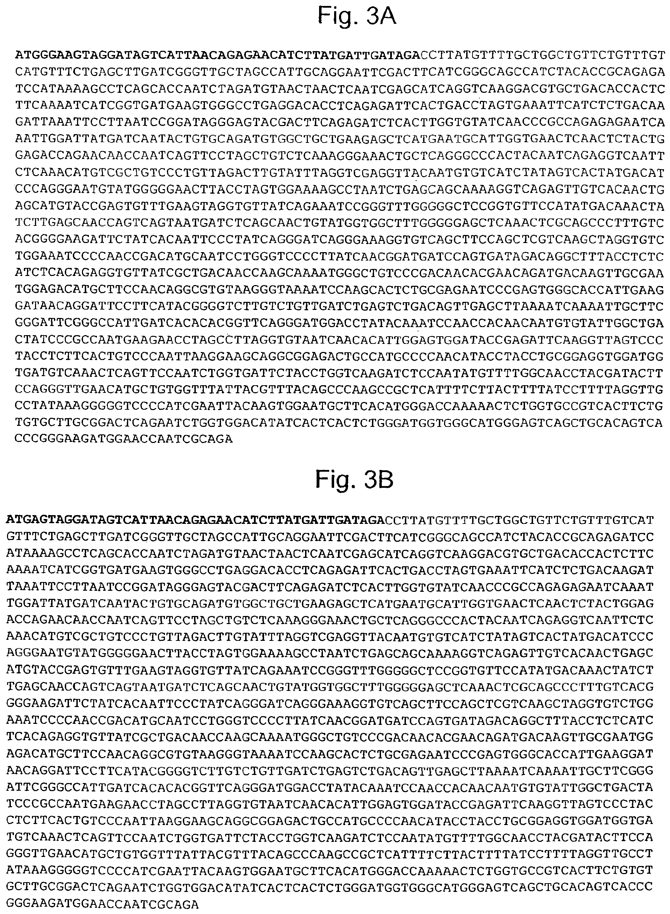

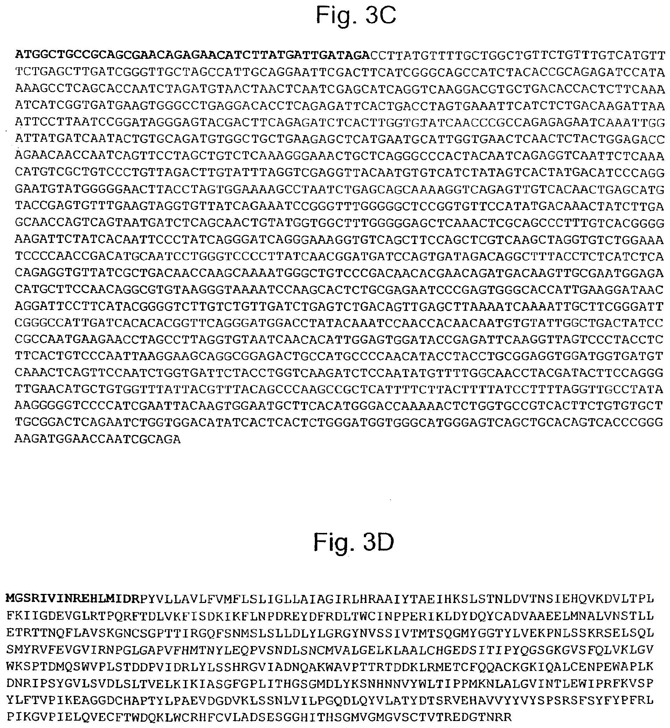

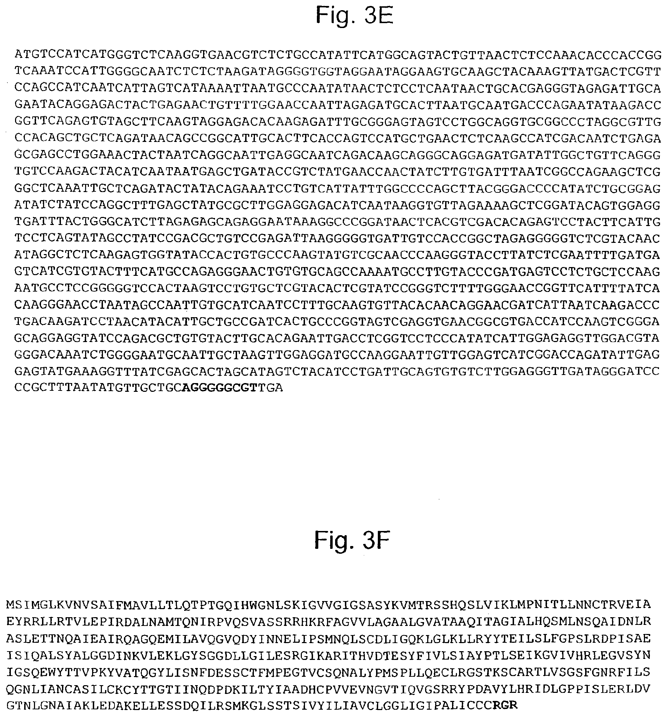

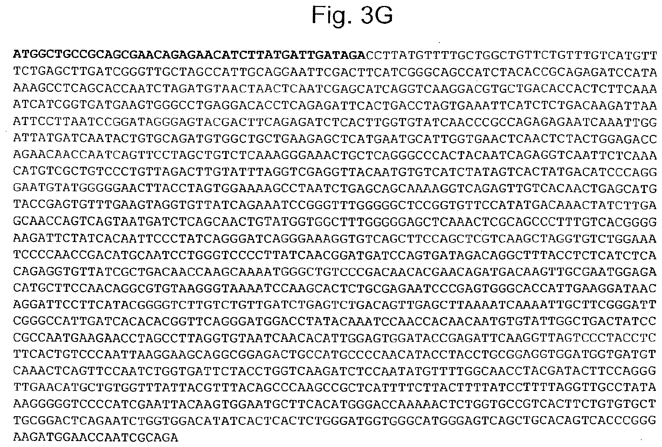

Figures 3A-3C exemplarily show the DNA sequence of three of the truncated H proteins derived from the wild-type (wt) DNA of the H protein of the Edmonton strain (H_{Edm}) of the measles virus (H_{Edm} wt DNA), namely, HcΔ18, HcΔ19 and HcΔ24+4A, respectively. Figure 3D exemplarily depicts the amino acid sequence of HcΔ18. Figures 3E and 3F exemplarily show the DNA and amino acid sequence of one of the truncated F proteins derived from the wild-type (wt) DNA of the F protein of the Edmonton strain of the measles virus (F_{Edm} wt DNA), namely FcΔ30. The cytoplasmic portions are shown in bold. Figure 3G depicts the DNA sequence of the truncated HcΔ24 protein, wherein additional four alanine residues were added to thus yield HcΔ24+4A. Again, the cytoplasmic portions are shown in bold.
Figures 4A and 4B provide an overview over exemplary generated F and H mutant proteins in comparison to their respective wt molecules, indicating the deleted (or added) amino acid residues in the cytoplasmic portion of the proteins.
Figure 5 depicts the amino acid sequence of the CD30ₒₚₜ.
Figure 6 shows exemplary microscopic pictures of iPSCs transduced with the pseudotyped lentiviral vector particles.
Figure 7 shows examples of embryoid bodies differentiated from CD30opt-LV transduced iPS cells (upper panel) or with a conventional lentiviral vector (VSVG-LV, bottom panel). The picture was taken three days after differentiation was started. The morphology shown in bright field (left panels) confirms embryoid body formation. The green fluorescence (right panels) confirms that the genetic modification introduced by the vector has been retained during differentiation.

### Detailed description of the invention

The inventors developed novel pseudotyped lentiviral vector particles comprising a morbillivirus fusion (F) protein and a mutated hemagglutinin (H) protein of the measles virus (MeV) or the Edmonton strain of the measles virus (MeV_{Edm}), wherein the cytoplasmic portions of the F and the H protein are truncated, and wherein the amino acids necessary for receptor recognition in the H protein are mutated that it does not interact with CD46, SLAM and/or nectin-4 and further has a single chain antibody to a cell surface marker of hESCs and iPSCs at its ectodomain.

Furthermore, the inventors found out that the *in vitro* targeted transduction of undifferentiated pluripotent hESCs and iPSCs is substantially improved by using these lentiviral vector particles pseudotyped with a truncated morbillivirus fusion (F) protein and a mutated and truncated morbillivirus hemagglutinin (H) protein further displaying a single chain antibody to a stem cell surface marker at its ectodomain.

The term "*in vitro*" refers to studies in experimental biology that are conducted using components of an organism that have been isolated from their usual biological context in order to permit a more detailed or more convenient analysis than can be done with whole organisms. In contrast, the term "*in vivo*" refers to work that is conducted with living organisms in their normal, intact state, while "*ex vivo*" refers to studies on functional organs that have been removed from the intact organism.

The term "stem cell" refers to a cell that has the ability to self-renewal, i.e. to go through numerous cycles of cell division while maintaining the undifferentiated state, and has potency, i.e. the capacity to differentiate into specialized cell types, e.g. a nerve cell or a skin cell.

According to the present invention, "undifferentiated pluripotent stem cells" or rather "pluripotent stem cells" (PSCs) are able to generate all cell types of the body, but not those of the placenta, which is derived from the trophoblast and associated structures. In a preferred embodiment of the present invention, the PSCs are embryonic stem cells (ESCs), more preferably human embryonic stem cells (hESCs). The hESCs may be obtained from single blastomers of human embryos without embryo destruction. They may also be derived from established cell lines, which are commercially available.
An alternative pluripotent stem cell type is derived from *in vitro* cultured somatic cells that have been reversed into an embryonic-like pluripotent state, i.e. induced pluripotent stem cells (iPSCs). The iPSCs are also able to generate all cell types of the body, but not the umbilical cord, trophoblasts and associated structures. They functionally and phenotypically resemble embryonic stem cells. There are several publications and patents describing the reprogramming technique and suitable markers (e.g. WO 2010/033084 A1, WO 2009/144008 A1). The reprogramming according to the present invention is performed by introduction of stem cell-associated genes into non-pluripotent, i.e. differentiated cells, preferably fibroblasts, either by viral transduction or transfection. The stem cell associated genes are selected from the group consisting of Klf-4, Sox-2, Oct4, Nanog, LIN-28 and c-Myc, wherein 3-6 factors are introduced, preferably using a cocktail of 4 factors (including Oct4, Sox-2, Nanog and LIN-28).

hES stem cell lines include H9 or HES-3. Preferable iPS stem cell lines include hCBEC (obtained via lentiviral gene transfer), hFFBiPS SB4 and hFFBiPS SB5 (obtained via "Sleeping Beauty" transposition), wherein the iPS stem cell lines of the present invention can be also generated from diseased patients, such as from patients having adenosine-deaminase deficiency-related severe combined immunodeficiency (ADA-SCID), Duchenne (DMD) and Becker muscular dystrophy (BMD), Parkinson disease (PD) or juvenile-onset type 1 diabetes mellitus (JDM). For gene therapy, these iPS stem cells have to be transduced with an intact copy of the defective gene and can then, upon differentiation into the relevant cell type, be transferred to patients without causing an immune response, as already described by Zhao, T. et al. (Nature, 2011, 474: 212-215). Furthermore, these iPS stem cells can be used as a model system for therapeutic approaches of gene therapy. After specific transduction of these iPS cells, the expression of the therapeutic gene can be monitored before differentiation and effects of different expression rates can be detected during differentiation. In a preferable embodiment of the present invention, iPS cells, derived from ADA-SCID patients, are transduced with the CD30-LV vector, thus, transferring the functionally active adenosine-deaminase (ADA) gene. The cells are then differentiated towards the haematopoietic lineage, wherein CD30-positive cells are collected and analysed for expression of ADA. These cells can then be transplanted into immunodeficient mice where they engraft and differentiate into all lineages of the haematopoietic system.

As used herein, the term "multipotent" refers to the ability to only differentiate into a limited number of types. For example, the bone marrow contains multipotent stem cells that give rise to all the cells of the blood but not to other types of cells. Multipotent stem cells are found in adult organisms, e.g. most organs of the body (e.g. brain, liver, heart) where they can replace dead or damaged cells.

"Lentivirus" (or "lentiviral") as used in the present invention is a genus of the *Orthoretroviridae,* a sub-family of the *Retroviridae,* wherein the viruses of the taxonomic family *Retroviridae* share, as a main feature, a positive stranded ssRNA genome (ss(+)RNA). The genetic information of these viruses is first reverse-transcribed from RNA into DNA by a reverse transcriptase provided by the virus and subsequently integrated into the host genome. Thus, the term "retro" refers to the activity of reverse transcriptase and the transfer of genetic information from RNA to DNA. As the viral genome is ss(+)RNA (mRNA) it can also directly be used by the cell as template for protein production.

According to the present invention, the lentivirus (LV) is selected from the group consisting of the human immunodeficiency virus (HIV), the bovine immunodeficiency virus (BIV), the feline immunodeficiency virus (FIV), the simian immunodeficiency virus (SIV) and equine infectious anemia virus (EIAV), more preferably HIV-1, HIV-2, SIV_{mac}, SIV_{pbj}, SIV_{agm}, FIV and EIAV, and even more preferably HIV-1.

With regard to the detailed description of elements of the lentivirus, reference is made to the corresponding definitions in WO 2008/037458 A2.

Three basic components on separate plasmids, for example on a protein expression plasmid (e.g. measles virus H or F protein expression plasmid), packaging plasmid (e.g. pCMVdR8.9), and/or transfer vector plasmid (e.g. pSEW), may be provided for generating lentiviral vector particles: a psi-negative *gag*/*pol* gene, a psi-negative env gene and a psi-positive expression vector. In another preferred embodiment, the psi-positive lentiviral expression vector and/or the psi-negative lentiviral *gag*/*pol* gene are derived from a lentivirus selected from the group consisting of HIV-1, HIV-2, SIV_{mac}, SIV_{pbj}, SIV_{agm}, FIV and EIAV, and even more preferably HIV-1.
"Lentiviral vector particles", "pseudotyped lentiviral vector particles" or rather "vector particles" as used in the present invention are replication deficient lentiviral vectors that are useful for transducing a nucleic acid sequence into a genome of a host or target cell. Examples of pseudotyped lentiviral vector particles are described in detail in WO 2008/037458 and EP 11 151 018.6 to which reference is made. Such vector particles only contain an incomplete genome of the lentivirus from which they are derived. In detail, the vector particles comprise a minimum of the Gag, Pol and Env proteins and an RNA molecule (which can be an expression vector). The Gag, Pol, and Env proteins that are needed to assemble the vector particle are derived from the retrovirus and are provided *in trans* by means of a packaging cell line, preferably human embryonic kidney 293 cells that contain the SV40 Large T-antigen (HEK-293T or 293T), human sarcoma cell line HT-1080 (CCL-121), lymphoblast-like cell line Raji (CCL-86), glioblastoma-astrocytoma epithelial-like cell line U87-MG (HTB-14), or T-lymphoma cell line HuT78 (TIB-161), more preferably HEK-293T. The RNA molecule or expression vector is usually derived from the genome of the original retrovirus, which means that it comprises all the necessary elements for an effective packaging into the resulting lentiviral vector particles (*inter alia* the psi element and long terminal repeats, LTRs). However, in order for the lentiviral vector particle to be replication deficient, the RNA molecule does not comprise the genetic information of at least one of the *gag, env,* or *pol* genes itself, wherein the genes are either removed or the expression of their gene products is prevented, preferably by frame shift mutation(s).

In another preferable aspect, the RNA molecule is not integrated into the host cell genome, due to a defective integrase or missing integration signals on the RNA. With this RNA the encoded protein is translated for a limited time within the transduced cell, facilitating differentiation into a certain lineage (Sarkis et al., Curr. Gene Ther. 2008, 8(6), pp. 430-437). Alternatively, no specific RNA is packaged, and the vectors can be used for specific protein transfer (Voelkel et al., PNAS USA 2010, 107(17), pp. 7805-7810).

Thus, in a preferable aspect, the RNA molecule of the lentiviral vector particle does not comprise the genetic information of all three of said genes, i.e. the *gag, env,* or *pol* genes. In a further embodiment, the RNA molecule does not comprise the genetic information of other nonessential genes, additionally. In case of HIV-1, these unessential genes are selected from the group consisting of *tat*, *vif*, *vpr*, *vpu* and *nef*. Instead, a normally heterologous nucleic acid sequence to be integrated into the target/host genome ("transgene", i.e. a gene to be transduced) is present in the expression vector that is under control of a suitable promoter, and is thus expressed upon integration of the gene into the genome of the host or target cell. The term "suitable promoters" according to the present disclosure refers to promoters, which regulate the expression of housekeeping genes that are constitutively expressed in any tissue, for example the EF1A1 gene encoding the α-subunit of the eukaryotic elongation factor 1 or the gene encoding the phospho-glycerat kinase, and which show only a mild down regulation upon stem cell differentiation. Thus, the promoter of the present disclosure can be selected from the group consisting of the cytomegalovirus (CMV)-promoter, the spleen focus forming virus (SFFV)-promoter, the elongation factor 1 alpha (EF1α)-promoter (the 1.2 kb EF1α-promoter or the 0.2 kb EF1α-promoter), the chimeric EF1α/IF4-promoter, and the phospho-glycerate kinase (PGK)-promoter, wherein the 1.2 kb EF1α-promoter and the SFFV-promoter are preferred.

In a further preferred embodiment, the lentiviral vector particle further comprises a psi-positive RNA expression vector comprising the transgene. Preferably, the psi-positive RNA expression vector comprises at least one selectable marker gene as the transgene.
In general, the terms "selectable marker" or "selectable marker gene", as used in the present invention, refers to a gene and its corresponding product that allow detection, selection and/or isolation of said product. If such a selectable marker is expressed by a cell, consequently such a cell or a population of such cells can be detected, selected and/or isolated. Selectable markers according to the present disclosure can be, *inter alia*, a molecule, preferably a peptide or protein, detectable by fluorescence, an enzyme catalyzing a reaction of which the resulting product is monitored, or a molecule that confers a resistance to, *inter alia*, an antibiotic. Detection, selection and/or isolation can be carried out, *inter alia*, by fluorescence activated cell sorting (FACS), a FACS cell sorter, use of fluorescence microscopy, use of immunofluorescence microscopy with primary and secondary antibodies, or by the use of antibiotics. Thus, in one preferred embodiment of the present invention said selectable marker gene is selected from the group consisting of a gene coding for GFP (green fluorescent protein), a gene coding for eGFP (enhanced GFP), a gene coding for an apoptosis-inducing protein, a gene coding for a cytotoxic protein, TNF-α gene, p53 gene, an interfering RNA, an interferon gene, the herpes virus thymidine kinase gene, and a gene coding for an immune stimulatory or therapeutic protein.

Thus, in a preferable embodiment, the lentiviral vector particles is based on a vector selected from the group consisting of HIV-1, HIV-2, SIV_{mac}, SIV_{pbj}, SIV_{agm}. FIV and EIAV, and even more preferably HIV-1. In a further embodiment, the RNA molecule of the lentiviral vector particle is based on the HIV-1 genome and comprises the LTRs, the psi element and the CMV promoter followed by the gene to be transduced, wherein the *gag, env, pol, tat, vif, vpr, vpu* and nef genes of HIV-1 are removed or expression of their gene products is prevented, and wherein the gene to be transduced is a selectable marker gene that is selected from the group consisting of a gene coding for GFP, a gene coding for eGFP, a gene coding for an apoptosis-inducing protein, a gene coding for a cytotoxic protein, TNF-α gene, p53 gene, an interfering RNA, an interferon gene, the herpes virus thymidine kinase gene, and a gene coding for an immune stimulatory or therapeutic protein.
For generating the lentiviral vector particles, the three basic components, usually provided on separate plasmids, are required: a psi-negative *gag*/*pol* gene, a psi-negative *env* gene and a psi-positive expression vector. Co-transfection of these components into a packaging cell line, i.e. a suitable eukaryotic cell line, for example HEK-293T, leads to the expression of the Gag, Pol and Env proteins as well as generation of psi-positive RNA. Since the *gag*/*pol* and *env* genes of the present disclosure are psi-negative, the mRNA generated prior to expression of the corresponding proteins is not assembled into the lentiviral vector particles. Contrasting this, the psi-positive RNA transcribed from the expression vector of the present disclosure is included into the resulting lentiviral vector particle.
Consequently, the replication deficient lentiviral vector particles of the present disclosure are generated comprising the Gag, Pol and Env proteins provided *in trans* by the packaging cell line, preferably HEK-293T, as well as the psi-positive RNA transcript of the expression plasmid lacking the genetic information for autonomous replication. However, as the lentiviral vector particles comprise the Gag, Pol and Env proteins, they are able to efficiently infect their target/host cells, reverse-transcribe their RNA, and integrate said genetic information into the genome of the target/host cell.
For further details and conditions reference is made to WO 98/037458 A2, in particular the Example part thereof.
The term "pseudotyped", "pseudotyped vector" or "pseudotyped vector particle", "pseudotyped lentiviral vector particle" as used in the present disclosure, refers to a vector particle bearing envelope glycoproteins derived from other viruses having envelopes. The host range of the lentiviral vectors or lentiviral vector particles of the present disclosure can thus be expanded or altered depending on the type of cell surface receptor used by the glycoprotein, since such vector particles possess the tropism of the virus from which the glycoprotein(s) were derived.

Thus, in one preferable aspect, the *env* gene of the pseudotyped lentiviral vector particle, that is originally derived from the same retrovirus as the *gag* and *pol* genes and as the RNA molecule or expression vector, is exchanged for the envelope protein(s) of a different enveloped virus. In a preferred embodiment of present invention, said envelope protein(s) are the fusion (F) and the hemagglutinin (H) glycoproteins of a paramyxovirus or a morbillivirus, preferably the measles virus (MeV), or the Edmonton strain of the measles virus (MeV_{Edm}).
The MeV_{Edm} uses one of the following three receptors for cell entry, namely, the protein known to be the regulator of complement activation factor, CD46 (Gerlier, D. et al., Trends Microbiol., 1995, 3: 338-345), SLAM (signaling lymphocyte-activation molecule; also known as CD150) or Nectin-4, wherein the H protein, a type II transmembrane protein, binds to the MeV receptors CD46, SLAM or Nectin-4 , and the F protein, a type I transmembrane protein, carries a hydrophobic fusion peptide that mediates membrane fusion upon receptor binding of H.
Thus, one known function of the F protein is mediating the fusion of viral membranes with the cellular membranes of the host cell. Functions attributed to the H protein include recognizing the receptor on the target membrane and supporting F protein in its membrane fusion function. The mechanism of MeV-induced membrane fusion may involve receptor-induced conformational changes in the H and then F proteins, suggesting a dynamic interaction between these two proteins during the transfection process.

According to the present invention as defined by the appended claims, the F and H proteins are "truncated" at their "cytoplasmic portions". The term "cytoplasmic portion", "cytoplasmic tail" or "cytoplasmic region", as used in the present invention, refers to the portion of the respective protein that is adjacent to the transmembrane domain of the protein and, if the protein is inserted into the membrane under physiological conditions, extends into the cytoplasm.
The term "truncated" generally refers to a deletion of amino acid residues of the designated protein, wherein "truncated" also refers to the corresponding coding nucleic acids in a nucleic acid molecule that codes for a given "truncated" protein. Furthermore, it is to be understood that the nucleic acid molecules encoding for a specific truncated or modified protein of the present invention are likewise encompassed, and *vice versa.* For example, this means that if a given protein is referred to, e.g. a truncated F protein such as FcΔ30, the nucleic acid encoding said protein is likewise encompassed.

In the present invention, reference is specifically made to "truncated H" or "truncated F" proteins, which designate the morbillivirus, preferably MeV H and F proteins, respectively, whose cytoplasmic portion has been truncated, i.e. amino acid residues (or coding nucleic acids of the corresponding nucleic acid molecule encoding the protein) have been deleted. "HcΔX" and "FcΔX" designate such truncated H and F proteins, respectively, wherein "X" refers to 1-50 residues, that have been deleted of the cytoplasmic portion, respectively, more preferably 25-30 residues for FcΔX and 10-25 residues for HcΔX. Thus, in a further and even more preferred embodiment the "truncated F protein" is FcΔ24 or FcΔ30 and/or the "truncated H protein" is selected from the group consisting of HcΔ14, HcΔ15, HcΔ16, HcΔ17, HcΔ18, HcΔ19, HcΔ20, HcΔ21+A, and HcΔ24+4A, more preferably HcΔ18, HcΔ19 or HcΔ24+4A.

In one preferable aspect, the truncated cytoplasmic portion of the F protein comprises at least one positively charged amino acid residue, wherein the truncated cytoplasmic portion of the H protein is truncated to allow efficient pseudotyping and has fusion support function. In a further preferred aspect, the truncated cytoplasmic portion of the H protein comprises at least nine consecutive amino acid residues of the C-terminal cytoplasmic portion of the H protein plus an additional methionine at the N-terminus. In another and even more preferred aspect, the truncated cytoplasmic portion of the F protein comprises at least three consecutive amino acid residues of the N-terminal cytoplasmic portion of the F protein and the truncated cytoplasmic portion of the H protein comprises at least 13 consecutive amino acid residues of the C-terminal cytoplasmic portion of the H protein plus an additional methionine at the N-terminus, wherein one to four of the N-terminal amino acid residues of said at least 13 consecutive amino acid residues of the C-terminal cytoplasmic portion of the H protein can be replaced by alanine residues. Furthermore, the cytoplasmic portion of the F protein of the present disclosure is located at the C-terminus of the protein.

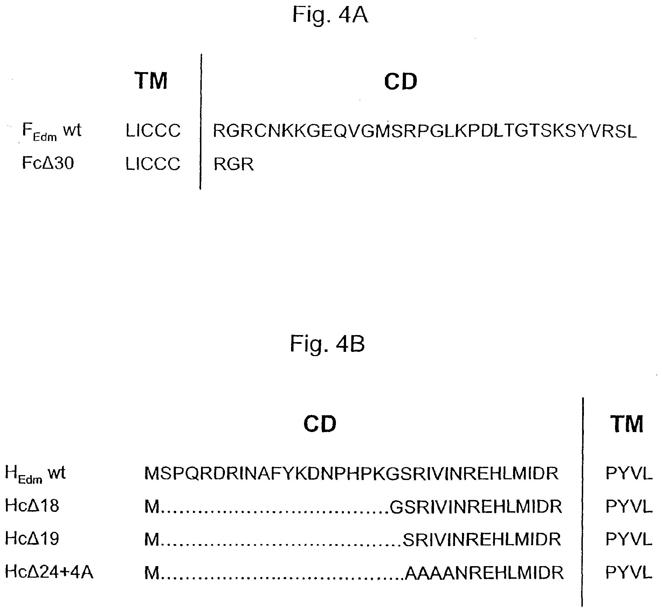

Thus, in a further and even more preferred aspect, the one or two psi-negative expression vectors) encode the truncated F protein FcΔ24 or FcΔ30 and/or the truncated H protein that is selected from the group consisting of HcΔ14, HcΔ15, HcΔ16, HcΔ17, HcΔ18, HcΔ19, HcΔ20, HcΔ21+A, and HcΔ24+4A, more preferably HcΔ18, HcΔ19 or HcΔ24+4A. In this regard reference is made to Figs. 3 and 4.
Normally, the lentiviral vector particles as pseudotyped with the morbillivirus F and H proteins can be used to efficiently transduce cells (or cell lines) that carry at least one of the three MeV receptors, CD46, SLAM, and nectin-4. However, according to the present disclosure, the H protein is a truncated and mutated ("HcΔX", "Hmut" or rather "HmutΔX") protein that does not interact with CD46,SLAM, and/or nectin-4. In one embodiment of the present invention, the mutation for ablating/preventing interaction of the H protein with CD46 is introduced by the point mutations Y481A and R533A of the MeV H protein. In another aspect, the Hmut protein also includes the mutations S548L and F549S, which lead to a more complete ablation of residual infectivity via CD46. Also, the mutation of the residues V451 and Y529 ablates productive interaction with CD46. Alternative mutations for ablating/preventing interaction of the H protein with CD46 relate to H proteins having Y481 replaced with any other amino acid, in particular with methionine or glutamine, or having mutations at one position selected from F431, V451, Y452, A527, P486, I487, A428, L464, G546, S548, F549 wherein these amino acids are replaced with another amino acid and this mutation prevents or assists in preventing interaction of the H protein with CD46. Altematively, replacement of all five consecutive residues 473 to 477 in H protein with alanine may prevent interaction of H protein with CD46. For preventing interaction of the H protein with SLAM one of the following residues may be replaced with any other amino acid, in particular, alanine: 1194, D530, Y553, T531, P554, F552, D505, D507. Furthermore, alternative mutations for ablating/preventing interaction of the MeV H protein with nectin-4 according to the present invention relate to the residues Y543 and P497, preferably Y543A or P497S and Y543A.

Thus, in a preferred aspect, the H protein contains at least one mutation for ablating/preventing interaction of the H protein with at least one of the CD46 receptors CD46, SLAM and/or nectin-4, preferably the H protein contains two or three muttations.

In one embodiment, the mutation for ablating/preventing interaction of the H protein with CD46 is a point mutation with another amino acid of at least one of the residues selected from the group consisting of V451, Y529, Y481, F431, V451, Y452, A527, P486, I487, A428, L464, R533, G546, S548, and F549, in particular with alanine, leucine, serin, methionine, glutamine, for example Y481A, R533A, S548L, and/or F549S; or Y481 replaced with methionine or glutamine. In an alternative embodiment, all five consecutive residues 473 to 477 are replaced with alanine in the H protein for ablating/preventing interaction of the H protein with CD46.

In another preferred embodiment, mutation for ablating/preventing interaction of the H protein with SLAM is a point mutation with another amino acid, preferably alanine, at at least one of the residues selected from the group consisting of 1194, D530, Y553, T531, P554, F552, D505, and D507.

In a further embodiment, the mutations for ablating/preventing interaction of the MeV H protein with nectin-4 relate to the residues Y543 and P497, preferably Y543A or P497S and Y543A.
Further details concerning the truncated F and H proteins and mutations can be derived from WO 98/037458 A2. Moreover, the person skilled in the art will readily be able to introduce mutations as, for example, additions and deletions, into a given nucleic acid or amino acid sequence. Such known methodologies are, for example, disclosed in Sambrook, J. et al., Molecular Cloning, A Laboratory Manual, 2nd ed., Cold Spring Harbor Laboratory (1989).
Furthermore, it has been found that the ratio between the amount of plasmid encoding the truncated F and H protein, respectively, used for the production of the pseudotyped lentiviral vector particles has a significant effect of the titers of the resulting vector particles. This means that an increased amount of plasmid coding for the truncated F protein compared to the amount of plasmid coding for the truncated H protein leads to both an increased titer and an increased transduction efficiency of the resulting pseudotyped lentiviral vector particle. In a preferred embodiment of the present disclosure the amount of the truncated F protein is 10-100%, 100-250%, 250-500%, 500-750%, 750-1000%, or more than 1000% higher and, preferably, 300% higher than the amount of truncated H protein.
The terms "titers" and "transduction efficiency" as used in the present invention refer to the ability of a vector particle to enter a cell and integrate the genetic information, in particular RNA information, into the genome of the cell. Thus, the terms "titer" and "transduction efficiency" can be used synonymously. The titer/transduction efficiency can, for example, be determined by the incorporation of a gene coding for a selectable marker, for example, under the control of a CMV promoter, into the expression vector of the vector particle. Upon transduction of such selectable marker by means of the vector particle, the transduced cells will then express the selectable marker. If the selectable marker is, for example, the GFP protein, the number of cells infected by a given number of vector particles can readily be determined, e.g. by FACS analysis, and directly corresponds to the titer or transduction efficiency of the vector particles that have been used for transduction. The titer or transduction efficiency of a given vector particle thus refers to the number of cells transduced relative to the number of vector particles used. Thus, a vector particle suspension having an "increased titer" or an "increased transduction efficiency" is able to transduce a higher number of cells at a given vector particle concentration than a different vector particle suspension having the same vector particle concentration. In a preferable embodiment of the present invention, the titer of the virus stock is used between 1 x 10⁶ and 1 x 10⁸ i.u./ml for the transduction of 1 x 10⁴ to 1 x 10⁶ iPS cells.

According to the present invention as defined by the appended claims, the mutated and truncated H protein further displays a single-chain antibody at its ectodomain (i.e. at the C-terminus), i.e. H-scFv wherein the single-chain antibody is directed against or binding to a "cell surface marker" or rather "cell marker" that refers to a molecule present on the surface of a stem cell. It represents a fusion construct or in other words a chimeric protein. Such molecules can generally be, *inter alia*, peptides or proteins that may comprise sugar chains or lipids, antigens, clusters of differentiation (CDs), antigens, antibodies or receptors. Since not all populations of cells express the same cell markers, a cell marker can thus be used to identify, select or isolate a given population of cells expressing a specific cell marker. Thus, the terms "cell entry targeted" or "targeted" lentiviral vector particles as used in the present invention refer to pseudotyped lentiviral vector particles, wherein the H protein is mutated and further has a single-chain antibody to a cell surface marker at its ectodomain, i.e. by fusing the single-chain variable fragment (scFv) anti-cell surface marker coding sequence to the coding sequence of the H protein. Therefore, the host range of a cell entry targeted lentiviral vector particles of the present invention is not expanded or altered depending on the tropism of the virus the H protein is derived from, but, depending on the specificity of the single chain antibody fused to the H protein.

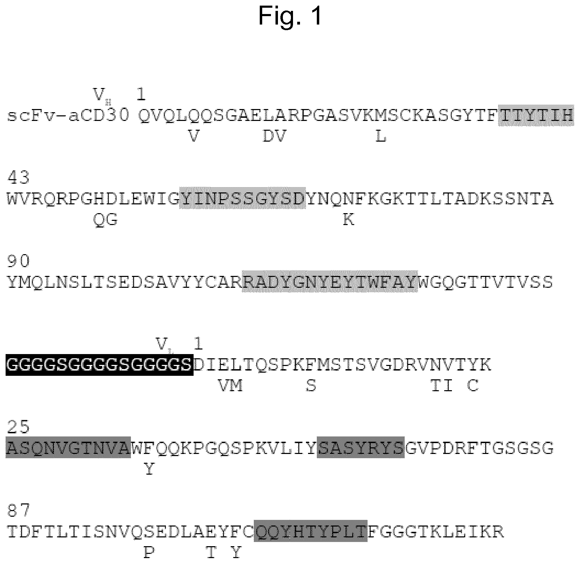

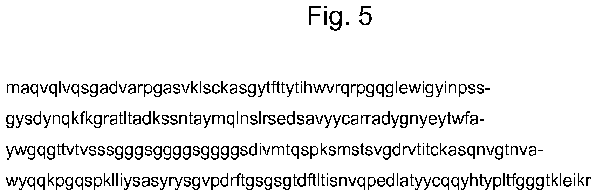

According to the present invention, the cell surface markers are selected from the group consisting of CD30, EpCAM (CD326), CD9, Thy-1 (CD90), SSEA-3, SSEA-4, TRA-1-60. and TRA-1-81. which are down regulated by differentiating stem cells. In a preferred embodiment, the mutated and truncated H protein further displays a single-chain antibody at its ectodomain that is directed against or binds to the cell surface marker CD30.
CD30 is a member of the tumour necrosis factor receptor family, originally identified as a surface marker for malignant cells in Hodgkin's disease (Schwab, U. et al., Nature, 1982, 299: 65-67). The biological function of CD30 in stem cells is still unknown, although its involvement in protecting stem cells against apoptosis was reported by Herszfeld, D. et al (Nat. Biotechnol., 2006, 24: 351-357). Furthermore, Grandela, C. & E. Wolvetang (Stem Cell Rev., 2007, 3: 183-191) observed that CD30-positive stem cells show a reduced expression of p53 (mediator or apoptosis) and an increased expression of *SURVIVIN* (inhibitor of apoptosis).
With regard to its expression, it has been reported that CD30 is strongly expressed in embryonal carcinomas (Pera, M. F. et al., Lab. Invest., 1997, 76: 497-504) but absent from most adult and embryonic tissue. Despite some contradicting results with respect to the CD30 expression in stem cells (Herszfeld, D. et al., Nat. Biotechnol., 2006, 24: 351-357; Grandela, C. & E. Wolvetang, Stem Cell Rev., 2007, 3: 183-191; Mateizel, I. et al., Human Reproduction, 2009, 24: 2477-2489) it is undisputed that CD30 expression is down-regulated during spontaneous differentiation of stem cells (Mateizel, I. et al., Human Reproduction, 2009, 24: 2477-2489; Lagarkova, M. A. et al., Cell Cycle, 2008, 7: 3610-3612). Thus, CD30 according to the present disclosure represents a marker of undifferentiated pluripotent hESCs and iPSCs.
It was found by the present inventors that the known scFvCD30 should be preferably mutated to improve surface expression and the titre of the produced targeted lentiviral vector particle, preferably by point mutations at the amino acid position 5, 11, 12, 20, 50, 51, and/or 70 in the V_{H} chain, more preferably Q5V, L11D, A12V, M20L, H50Q, D51G, and/or N70K; and at the amino acid positions 3, 4, 10, 20, 21, 23, 36, 98, and/or 104, more preferably E3V, L4M, F10S, N20T, V21I, Y23C, F36Y, S98P, E103T, and/or F105Y in the V_{L} chain. The amino acid sequence of the scFv-antiCD30 and the corrected/optimized amino acid sequence are shown in Fig. 1 and 5.

The Epithelial Cell Adhesion Molecule (EpCAM) is a homophilic cell adhesion protein, mainly expressed on strongly replicating epithelia, like mucosa or basal skin layer. During embryogenesis, it is expressed on precursors of e.g. hepatocytes (De Boer, C. J. et al., J. Pathol., 1999, 188: 201-206) but not on adult cells. Its *de novo* expression on all carcinoma (Roovers, R. C., et al., Br. J. Cancer, 1998, 78: 1407-1416) is associated with dedifferentiation and enhanced proliferation of the tumour cells. Furthermore, the expression of EpCAM is associated with the maintenance of the undifferentiated phenotype of hES cells (Lu, T.-Y., et al., J. Biol. Chem., 2010 285: 8719-8732). Thus, EpCAM according to the present disclosure represents a marker of undifferentiated pluripotent hESCs and iPSCs.
The scFv recognising EpCAM is described by (Willuda, J. et al., Cancer Res., 1999, 59: 5758-5767). The H-scFv fusion protein cloning according to the present disclosure can be performed following to the procedure already described by Anliker, B. et al. (Nat. Methods, 2010, 7: 929-935), wherein the specific transduction of EpCAM-positive cells proves target specificity and appropriate titers.
CD9 (TG30) is a tetraspanin and described to be involved in cell adhesion (Masellis-Smith, A. & A. R. Shaw, J. Immunol., 1994, 152: 2768-2777), motility and metastasis (Ikeyama, S. et al., J. Exp. Med., 1993, 177: 1231-1237). Furthermore, it has a role in egg/sperm fusion (Higginbottom, A. et al., Biochem. Biophys. Res. Commun., 2003, 311: 208-214) and is listed by "The International Stem Cell Initiative" (Nat. Biotechnol., 2007, 25: 803-816) as marker for hES cells. Thus, CD9 is a cell surface protein known to function during cell development, growth and motility and is down regulated by differentiating hPSCs, whereby CD9 according to the present disclosure represents a marker of undifferentiated pluripotent hESCs and iPSCs.
Thy-1 (CD90) is a singlepass transmembrane protein with an Ig-like V-type (immunoglobulin-like) domain and is also described to be expressed on hematopoietic precursor cells (Craig, W. et al., J. Exp. Med., 1993, 177: 1331-1342). Thus, CD90 can be considered as a surrogate marker for various kinds of stem cells (e.g. hematopoietic stem cells or hES cells, including undifferentiated pluripotent hESCs and iPSCs Stage-specific embryonic antigens 3 and 4 (SSEA-3 and SSEA-4) are two different epitopes on the same glycolipid exclusively present on hES or iPS cells (Thomson, J. A. et al., Science, 1998, 282: 1145-1147) or embryonal carcinoma cells, which are down regulated upon differentiation (Draper, J.S. et al., J. Anat., 2002, 200(Pt 3): 249-258). Thus, SSEA-3 and SSEA-4 according to the present disclosure represent marker of undifferentiated pluripotent hESCs and iPSCs.
In summary, the pseudotyped lentiviral vector particles, with a truncated F protein (for example FcΔ30) and a mutated H protein (e.g. "HmutΔ18) additionally displaying a single-chain antibody to a cell surface marker at its ectodomain, for example scFvCD30, scFvEpCAM (scFvCD326), scFvCD9, scFvThy-1 (scFvCD90), scFvSSEA-3, scFvSSEA-4, TRA-1-60, or TRA-1-81, no longer enter cells via CD46,SLAM, and/or nectin-4, but are rather targeted to and enter only those cells displaying the respective corresponding markers at their surface. This means that the titers are significantly reduced on cells not expressing these markers. Therefore, cell entry and transduction using the pseudotyped lentiviral vector particles of the present disclosure represent efficient and effective means for highly selective gene transfer into specific cells, i.e. for the targeted transduction of specific cells. According to the present disclosure such cells are stem cells, more preferably undifferentiated pluripotent stem cells, wherein the stem cells are selected from the group consisting of hESCs and iPSC.

The term "differentiation" or "differentiation pathway" as used in the present disclosure refers to the pathway followed by a stem cell as it heads towards a lineage-specific or mature cell type and so begins with an undifferentiated stem cell and ends with a lineage-specific or mature, differentiated cell. A stem cell following such a pathway can go through many stages, wherein "undifferentiated pluripotent stem cells" or rather "pluripotent stem cells" are thought to have the potential to differentiate in almost any cell type, while "undifferentiated multipotent stem cells" or rather "multipotent stem cells" are believed to have the potential to differentiate into many cell types only. Since CD30, EpCAM (CD326), CD9, Thy-1 (CD90), SSEA-3, SSEA-4, TRA-1-60, and TRA-1-81 expression is undetectable in the areas of differentiation, CD30, EpCAM (CD326), CD9, Thy-1 (CD90), SSEA-3, SSEA-4, TRA-1-60, and TRA-1-81 expression reflects the undifferentiated state of the stem cells. Thus, the pseudotyped lentiviral vector particles of the present disclosure specifically transduce (i.e. targeted transduction) undifferentiated pluripotent CD30-, EpCAM (CD326)-, CD9-, Thy-1 (CD90)-, SSEA-3-, or SSEA-4-, TRA-1-60-, and TRA-1-81-positive stem cells, more preferably human embryonic stem cells (hESCs) and induced pluripotent stem cells (iPSCs), wherein present targeted transduction of these cells does not interfere with their pluripotency. Thus, depending on the transgene of the present disclosure transgene silencing may be prevented which occurs over time or upon stem cell differentiation.
To summarize, the present invention is based on the unexpected and surprising finding that the incorporation of morbillivirus, preferably MeV, F and H proteins having truncated cytoplasmic tails into lentiviral vector particles, and the complex interaction of these two proteins during cellular fusion, allows for a superior and more effective transduction of cells, as the measles virus is characterized by direct and high efficient membrane fusion. Moreover, these pseudotyped vector particles allow the targeted gene transfer or rather targeted transduction of CD30-, EpCAM (CD326)-, CD9-, Thy-1 (CD90)-, SSEA-3-, SSEA-4-, TRA-1-60, and TRA-1-81-positive stem cells, more preferably human embryonic stem cells (hESCs) and induced pluripotent stem cells (iPSCs), as CD30, EpCAM (CD326), CD9, Thy-1 (CD90), SSEA-3, SSEA-4, TRA-1-60, and TRA-1-81 expression is rapidly down-regulated upon differentiation of hESCs and iPSCs. Thus, the titres and the transduction efficiency of the pseudotyped lentiviral vector particles of the present disclosure are significantly increased and only multipotent stem cells are transduced, so that the transgenes are not silenced upon stem cell differentiation. Targeting the molecule CD30, EpCAM (CD326), CD9, Thy-1 (CD90), SSEA-3, SSEA-4, TRA-1-60, or TRA-1-81 with CD30-, EpCAM (CD326)-, CD9-, Thy-1 (CD90)-, SSEA-3-, SSEA-4-, TRA-1-60, or TRA-1-81-specific lentiviral vectors is very efficient and strictly specific. As shown in the example part, it was possible with this approach that only iPSC colonies expressing Oct4 (a well known stem cell marker) express the marker gene (e.g. GFP) after transduction with CD30-, EpCAM (CD326)-, CD9-, Thy-1 (CD90)-, SSEA-3-, SSEA-4-, TRA-1-60, or TRA-1-81-lentoviral vector particles. Other stem cell makers can be included in the embodiments of the present disclosure, e.g. Sox-2, Nanog as well as expression and activity of alkaline Phosphatase (an enzyme, which is only active in pluripotent cell types). This is an huge advantage since the prior art transduction technologies cannot discriminate stem cells neither from feeder cells nor from differentiated cells, as they normally occur during the cultivation of iPSCs.

The present disclosure is particularly suitable for basic research, e.g. functional genomics, as well as therapeutic applications of iPSCs. The invented method is faster, easier to handle and gentler for the cells than state of the art protocols. Moreover, fewer cells will be required. With regard to iPSCs which are a non-homogenous mixed cell population composed of pluripotent cells, differentiated and undifferentiated cells it is an advantage that with the present disclosure it is possible to distinguish between cells expressing certain markers (e.g. CD30, EpCAM (CD326), CD9, Thy-1 (CD90), SSEA-3, SSEA-4, TRA-1-60 or TRA-1-81) and to selectively and specifically transduce these cells, wherein present targeted transduction does not interfere with the pluripotency, i.e. present transduced hESCs and iPSCs are able to differentiate into cell types of all three germ layers which can be tested using different germ layer marker genes, for example β-tubulin or cytokeratin-18 for ectoderm; α-fetoprotein (AFP), sex determining region Y-box 17 (SOX-17) or hepatocyte nulear factor-4 (HNF4) for endoderm; desmin or CD31 for mesoderm.

The invention is further described in the following Examples. Examples not falling within the scope of the claims are for illustrative purposes only.

### Examples

### Example 1: Production and Isolation of pseudotyped lentiviral vector particles displaying scFV-CD30

As described in WO/2008/037458, the scFv anti-CD30 coding sequence was genetically fused to the modified coding sequence of measles virus hemagglutinin (H) protein. The scFv framework was modified as described in the previous patent application EP2476754. Clone pHL3-CD30mut2#2 mediated a high titer and was used for further experiments. The transfection method for the production of vector particles is described in Anliker, B. et al. (Nat. Methods, 2010, 7: 929-935). Briefly, HEK-293T cells were transfected using poly-ethylene-imine (PEI). For this purpose, 1.5 × 10⁷ cells were seeded 24 h before transfection into a T175 flask. Before transfection, the medium was replaced with 15 ml of Dulbecco's modified Eagle medium (DMEM) supplemented with 2 mM glutamine. In total, 2.6 µg of measles virus H protein expression plasmid, 8 µg of measles virus fusion (F) protein expression plasmid, 28.7 µg of packaging plasmid pCMVdR8.9 and 30.2 µg of transfer vector plasmid pSEW were mixed with 310 µl of 5% (wt/vol) glucose. In parallel, 70 µl of 18 mM PEI and 310 µl of 5% glucose were mixed. Both mixtures were incubated for 10 min at room temperature (25°C), combined vortexed and incubated for additional 10 min at room temperature. After adding 1 ml DMEM supplemented with 2 mM glutamine, the mixture was added to the cells. After 4 h, 2 ml of FCS were added. The cell supernatant containing the pseudotyped particles was filtered 48 h later (0.45-µm filter) and concentrated by ultracentrifugation over a 20% (wt/vol) sucrose cushion (100,000 g for 3 h at 4°C). The supernatant was discarded and the pellet was resuspended in 150 µl reduced serum medium (Opti-MEM) or phosphate buffered saline (PBS). Vector particle stocks were titrated on HuT78 cell line. eGFP expressing cells (eGFP⁺ cells) were determined by FACS analysis and titers were calculated.

Similar experiments were carried out using clone pHL3-CD30ₒₚₜ.which showed similar good titers as the above mentioned CD30mut#2.

### Example 2: Cultivation and Transduction of iPSCs

iPS cells are maintained on an inactivated feeder layer of murine embryonic fibroblasts in the presence of 20% Knock-Out (KO) Serum replacement, 1 mM non-essential amino acids, 1 mM L-glutamine, 0.1 mM β-mercapto-ethanol, 50 U penicillin/50 µg streptomycin (Pen/Strep) and 8 ng/ml basic fibroblast growth factor (bFGF or FGF2) in KO-DMEM. For subcultivation, medium was removed and collagenase (2 mg/ml Collagenase Type IV in KO-DMEM) was added for 5 min to dissolve the stem cell colonies from the feeder layer. Subsequently, 1 ml medium was added to wash the colonies from the feeder layer and the colonies were concentrated by centrifugation (3 min, 800 rpm, 4°C). The cell pellet was resuspended in an appropriate amount of fresh medium and allocated to fresh feeder plates.

For the transduction of iPS cells, an appropriate amount of colonies was seeded two days before transduction. To 1 ml cell culture medium, 10 µl of vector stock (titer: 1 x 10⁷ i.u./ml) was added resulting in a multiplicity of infection (MOI) of 2 when 5 x 10⁴ iPS cells are present in the well. Alternatively, the vector stock was added to the cells before allocation to the fresh feeder plates.

After transduction, the cells were cultivated for another 4 days until GFP expression derived from the transferred reporter gene became detectable. A reasonable number of transduced cells were already detectable after adding the CD30-LV directly to the cultured cells. Cells were fixed with 4% formaldehyde in PBS at room temperature for 15 min for immunofluorescence analysis. The marker Oct4 was visualised in parallel to the GFP expression by an Oct3/4 antibody from Santa Cruz (incubation at room temperature for one hour) and a secondary goat-anti-mouse-antibody (Alexa-647; 1:1000) after an incubation at room temperature for 30 min.

### Example 3: Cell entry targeted lentiviral vector particles directed to CD30-expressing cells

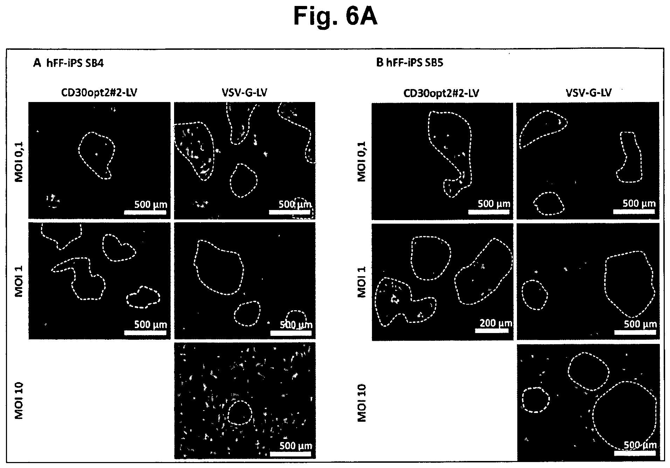

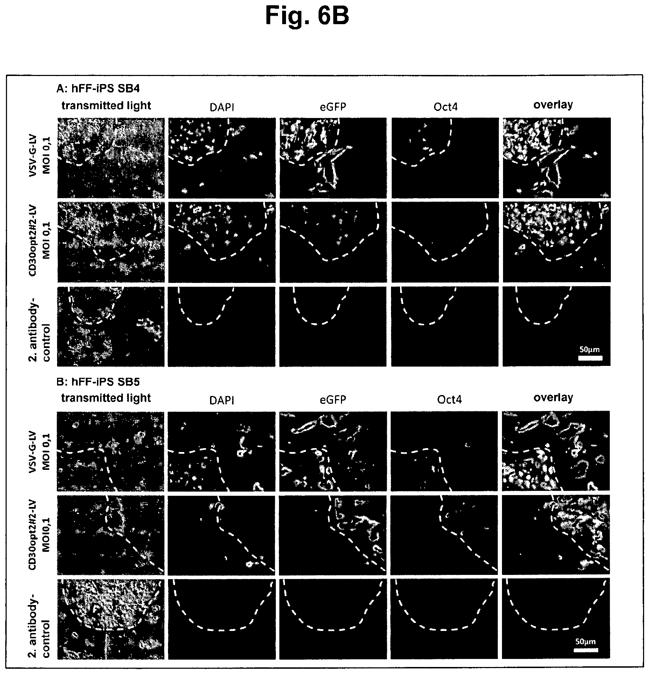

1.6 x 10⁵ of the iPS cell lines hFFBiPS SB4 and hFFBiPS SB5 were seeded on a feeder layer of mouse embryo fibroblasts in a 24-well-plate. Transduction followed 24 h later with vesicular stomatitis virus G protein (VSV-G) pseudotyped lentiviral vectors (VSV-G-LV) and CD30ₒₚₜ-LV (MOI 0.1; 1 and 10 [only VSV-G-LV]) in 500 µl stem cell medium. After 6 h, medium was changed and cells were cultivated for 120 h. The eGFP-expression of transduced cells was analysed by fluorescence microscopy (Figure 6A). For immunofluorescence pictures showing Oct4, CD30 or SSEA-4, cells were seeded on coverslips and transduced at an MOI of 0.1. Then the cells were washed with PBS once, fixed for 15 min with 4% formaldehyde in PBS and permeabilised with 1% Triton X-100 for 10 min at room temperature (RT). Unspecific binding sites were blocked with 5% bovine serum albumin (BSA)/0.1% Triton X-100 in PBS for 30min at RT. The primary antibodies were used 1:500 in PBS with 5% BSA and incubated with the cells for 1 h at RT. Cells were washed three times with PBS for 5 min then incubated with the according secondary antibodies (diluted 1:1000 in PBS) for 30 min at RT in the dark. After washing with PBS (three-times, 5 min) nuclei were stained with 4'6-diamidino-2-phenylindole (DAPI) (1 µg/ml in PBS) for 1 min and washed again three-times for 10 min. Coverslips were mounted on slides with Aqua-polymount (Polysciences Europe GmbH, Eppelheim, Germany) and stored at 4°C until analysis.

### Example 4: Different promotors suitable for transgene expression after differentiation

Transduction of iPS cells with lentiviral targeting vectors should not interfere with the pluripotency of the cells. Therefore, transduced cells must be demonstrated to differentiate into all germ layer lineages. This can be demonstrated by embryoid body formation in cell culture, or teratoma formation after injection into immundeficient mice. Besides the SFFV-promoter that is silenced during differentiation, four different promoters have been tested: the 1.2 kb EF1α-promoter, the chimeric 0.4 kb EF1α/IF4 promoter, the short 0.2 kb EF1α-promoter and the PGK-promoter. To test the expression of a marker gene under control of these different promoters, 1 x 10⁵ cell lines derived from different human tissues (293T, HT1080, Raji, U87-MG and HuT78) have been transduced with VSV-G-LV at an MOI of 0.1 and analyzed GFP-expression after 96 h. In all cell lines, the 1.2 kb EF1α-promoter showed a similar strong activity as the SFFV-promoter and was used for iPS cell transduction.

### Example 5: Differentiation of iPS to embryoid bodys and lineages of all three germlayers after marker gene transfer

According to a protocol of Cerdan, C. et al. (Curr. Protoc. Stem Cell Biol. 2007, 1:1D.2.1-1D.2.16), iPS cells were grown in six-wells on the feeder-layer in 3 ml embryonic stem cell (ESC) medium (40 ml KnockOut-DMEM, 10 ml KnockOut Serum Replacement, 0.5 ml MEM non-essential amino acids, 0.25 ml L-glutamine, 0.25 ml Pen/Strep, 0.1 µl 2-mercaptoethanol, 4 ng/µl FGF2) until they form colonies and express Oct4 uniformly. When the iPS cells were ready for subcultivation, differentiation was started. To induce the formation of embryoid bodies (EBs), iPS cells in one six-well plate were detached from the feeder using 1 mg/ml collagenase (Type IV). For this purpose, the medium was aspirated, 1 ml collagenase was added to each well and the cells were incubated for 5 min at 37°C. 2 ml ESC medium was added to each well and the colonies were carefully detached from the feeder using a 1 ml pipette.

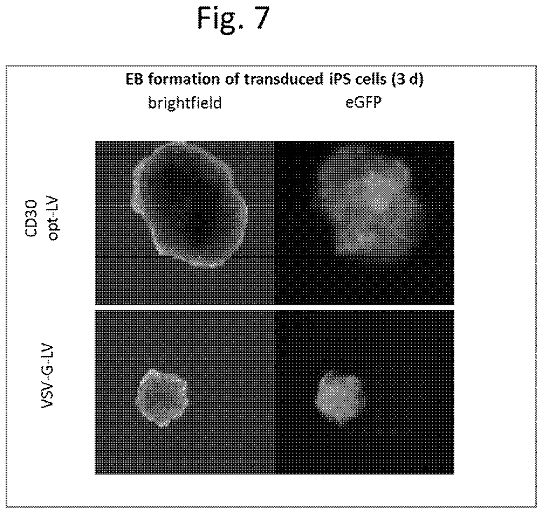

The colonies were transferred to a 15 ml tube and centrifuged for 3 min at 800 rpm. The supernatant was aspirated and the iPS cells were resuspended in EB medium (80 ml KnockOut DMEM, 20 ml FCS, 0.5 ml L-glutamine, 0.67 µl 2-mercaptoethanol, 1 ml ascorbic acid (5 mg/ml), 0.67 ml holo-transferrin (30 mg/ml). 3 ml of cell suspension were transferred to ultra-low attachment plates and incubated at 37°C. Medium was exchanged every 2 days. The general formation of EBs was stopped after 10 days of differentiation and expression of GFP was monitored. Figure 7 shows examples of embryoid bodies differentiated from CD30opt-LV transduced iPS cells (upper panel) or with a conventional lentiviral vector (VSVG-LV, bottom panel). The picture was taken three days after differentiation was started. The morphology shown in bright field (left panels) confirms embryoid body formation. The green fluorescence (right panels) confirms that the genetic modification introduced by the vector has been retained during differentiation.
To monitor the expression of marker genes for all three germ layers RNA was extracted at different time points during differentiation and reverse transcriptase (RT)-PCR performed with primer pairs specific for different germ layer marker genes (ectoderm: β-tubulin, cytokeratin 18; endoderm: AFP, SOX-17, HNF4; mesoderm: desmin, CD31).

To induce differentiation of iPS cells to spinal motoneurons a protocol published by Hu, B. Y. & S. C. Zhang (Nat. Protoc., 2009, 4:1295-1304) is applied. For the differentiation the following steps have been used: Induction of primitive neuroectoderm (NE) cells (days 0-10), specification of the basic helix-loop-helix transcription factor Olig2-expressing motoneuron progenitors (days 10-28), generation of spinal motor neurons by differentiating on the substrate (days 28-35) and maturation of spinal motor neurons on the substrate (days 42-56). For this purpose, iPS cells were detached from the mouse embryonic fibroblast (MEF) feeder layer using collagenase type IV, collected in a 15 ml tube and centrifuged for 1 min at 50 g and room temperature. The colonies were then transferred to T75 flasks and cultured at 37°C in ESC medium without FGF2. Medium was exchanged every second day. On day 7 medium was exchanged for neural differentiation medium (484 ml DMEM medium with nutrient mixture F-12 (DMEM/F12), 5 ml of serum free supplement based on Bottenstein's N-1 formulation (N-2 supplement), 5 ml MEM non-essential amino acids, 5 ml Pen/Strep, 1 ml heparin (1 mg/ml)). 20-25 clusters (EB's) were seeded on a laminin-coated 12-well plate in 300 µl neural differentiation medium containing retinoic acid (0.1 µM) and sonic hedgehoc homolog (SHH) protein (100 ng/ml). The following day 2 ml of neural differentiation medium was added and the clusters cultured at 37°C. On day 10 medium was changed to neural differentiation medium containing retinoic acid (0.1 µM). On day 15 neural tube-like rosettes were carefully detached from the plate, transferred to a new cell culture T75 flask containing neural differentiation medium and retinoic acid. Medium was exchanged every second day. On day 20 the clusters were transferred to a 15 ml tube and centrifuged for 2 min at 50 g and room temperature. After removal of the medium the clusters were incubated with 1 ml Accutase until the suspension looks cloudy. After addition of 9 ml neural differentiation medium the cells were pelleted and the clusters were downsized by careful pipetting. The smaller clusters were transferred to a new cell culture T75 flask. Medium was exchanged every second day. On day 28 generation of spinal motor neurons was induced by seeding 3-5 spheres on poly-L-ornithin/laminin coated plates in the presence of brain-derived neutrophic factor (BDNF), glial cell line-derived neutrophic factor (GDNF), insulin-like growth factor 1 (IGF-1) (10 ng/ml), cAMP (1µM), ascorbic acid (200 ng/ml), retinoic acid (50 nM) and SHH (50 ng/ml). Medium was exchanged every second day until day 56. On day 35 some cells were stained for the presence of HB9. On day 42 the expression of choline acetyltransferase (ChAT) was evaluated. On day 56 immunostaining for synapsin 2 was performed.

To generate hepatocytes, a protocol published by Moore, R. N. & P. V. Moghe (Stem Cell Res., 2009, 3: 51-62) was used. Briefly, the iPS cells were differentiated by cultivation on MEFs or Matrigel-coated plates in the presence of various growth factors over a 2-weeks period. For the differentiation, cells were grown in stem cell medium without FGF2 but supplemented with dexamethasone (10-7 M), oncostatin M (10 ng/ml), hepatocyte growth factor (20 ng/ml), activin A (50 ng/ml) and Wnt 3A (100 ng/ml) for two weeks with daily exchange of the medium. To determine the extent of differentiation the cells were stained for hepatospecific markers (AFP, albumin, cytokeratin 18 (CK18), alpha-1-antitrypsin (AAT), HNF-4, forkhead box A2 (FOXA2), inducible cytochrome P450 1A2 (CYP1A2)) or analysed by RT-PCR. Pure populations of hepatocytes were generated by FACS sorting. Functionality of the hepatocytes was determined by an albumin secretion analysis using an enzyme-linked immunosorbent assay (ELISA assay) as well as by an ethoxyresurofin-O-deethylase (EROD) assay to monitor CYP1A2 activity via the conversion of ethoxyresorufin to resorufin according to the method described in Moore, R. N. & P. V. Moghe (Stem Cell Res., 2009, 3: 51-62).

For the generation of cardiomyocytes, a protocol adapted from Laflamme, M. A. et al. (Nat. Biotechnol., 2007, 25: 1015-1024) was used. For the induction of cardiac differentiation pluripotent cells were plated on Matrigel-coated plates and cultivated in RPMI1640 plus B27 supplement (RPMI-B27) medium including 100 ng/ml human recombinant activin A for 24h, followed by 10 ng/ml human recombinant bone morphogenic protein 4 (BMP4) for 4 days. Then the medium was changed to RPMI-B27 without supplementary cytokines for 2-3 additional weeks with medium exchange every second day. To determine the differentiation status of the cells, RT-PCR analysis of several cardiac mesodermal marker genes (transcription factor GATA-4, homeobox protein-coding gene Nkx2.5, T-box transcription factor-coding genes Tbx5 and Tbx20; myosin light chain (MLC) kinases Mlc2a and Mlc2v) was performed. The expression of atrial natriuretic factor (ANF), myosin light chain 2 (MIc2), myosin heavy chain (MHC), troponin T, Titin, ANF and sarcomeric α-actinin were evaluated by immunofluorescence staining. Pure populations of cardiomyocytes were isolated by FACS sorting.

## Claims

1. Use of a pseudotyped lentiviral vector particle in the in-vitro targeted transduction of undifferentiated pluripotent human embryonic stem cells (hESC) and induced pluripotent stem cells (iPSC), said vector comprising a morbillivirus fusion (F) protein and a mutated hemagglutinin (H) protein of the measles virus (MeV) or the Edmonton strain of the measles virus (MeV_{Edm}),
wherein the cytoplasmic portions of the F and the H protein are truncated and the truncated F protein is FcΔ24 (24 amino acids removed from C-terminus) or FcΔ30 (30 amino acids removed from C-terminus) and the mutated and truncated H protein is selected from the group consisting of HcΔ14 (amino acids 2-15 removed), HcΔ15 (amino acids 2-16 removed), HcΔ16 (amino acids 2-17 removed), HcΔ17 (amino acids 2-18 removed), HcΔ18 (amino acids 2-19 removed), HcΔ19 (amino acids 2-20 removed), HcΔ20 (amino acids 2-21 removed), HcΔ21+A (amino acids 2-22 replaced by one alanine) and HcΔ24+4A (amino acids 2-25 replaced by four alanines), preferably HcΔ18, HcΔ19, and HcΔ24+4A,
wherein the amino acids necessary for receptor recognition in the H protein are mutated so that it does not interact with CD46, SLAM and/or nectin-4 and wherein the H protein further has a single chain antibody directed against CD30, EpCAM (CD326), CD9, Thy-1 (CD90), SSEA-3, SSEA-4, TRA-1-60, or TRA-1-81 at its ectodomain.

2. The use according to claim 1, wherein the mutation in the H protein is:
- at least one point mutation with another amino acid at the residues selected from the group consisting of V451, Y529, Y481, F431, V451, Y452, A527, P486, 1487, A428, L464, R533, G546, S548, and F549 with alanine, leucine, serin, methionine, or glutamine; and/or
- a point mutation of all five consecutive residues 473 to 477 with alanine; and/or
- at least one point mutation with another amino acid at the residues selected from the group consisting of 1194, D530, Y553, T531, P554, F552, D505, and D507; and/or
- at least one point mutation with another amino acid at the residues selected from the group consisting of Y543 and P497.

3. The use according to any of claims 1 to 2, wherein the scFv is directed against CD30 having the optimized amino acid sequence as shown in Fig. 5.

4. The use according to any of claims 1 to 3, wherein the pseudotyped lentivral vector particle is derived from a lentivirus selected from the group consisting of HIV-1, HIV-2, SIV_{mac}, SIV_{pbj}, SIV_{agm}, FIV and EIAV, preferably HIV-1.

5. The use according to claim 4, wherein the pseudotyped lentiviral vector particle does not comprise the genetic information of the *gag*, *env*, and/or *pol* genes and/or of other nonessential genes, which are selected from the group consisting of *tat*, *vif*, *vpr*; *vpu*, and *nef.*

6. The use according to claim 5, wherein said genes are provided in trans by means of a packaging cell line, which is selected from the group consisting human embryonic kidney 293 cells that contain the SV40 Large T-antigen (HEK-293T or 293T), human sarcoma cell line FIT-1080 (CCL-121), lymphoblast-like cell line Raji (CCL-86), glioblastoma-astrocytoma epithelial-like cell line U87-MG (HTB-14), and T-lymphoma cell line HuT78 (TIB-161), more preferably HEK-293T.

7. The use according to any of claims 1 to 6, wherein the amount of the truncated F protein present in said vector particle is higher than the amount of mutated and truncated H protein present in said vector particle, preferably 10-100%, 100-250%, 250-500%, 500-750%, 750-1000%, or more than 1000% higher and, preferably, 700% higher than the amount of mutated and truncated H protein.

8. The use according to any of claims 1 to 7, further comprising a psi-positive RNA expression vector, wherein the psi-positive RNA expression vector comprises at least one selectable marker gene.

9. The use according to claim 8, wherein the selectable marker gene is selected from the group consisting of a gene coding for GFP, a gene coding for eGFP, a gene coding for an apoptosis-inducing protein, a gene coding for a cytotoxic protein, TNF-α gene, p53 gene, an interfering RNA, an interferon gene, the herpes virus thymidine kinase gene, a gene coding for an immune stimulatory protein and a gene coding for a therapeutic protein.

10. Use according to any of claims 1-9, wherein the iPSCs are selected from the group consisting of hFFBiPS SB4 and HFFBiPS SB5 or are generated from patients having adenosine-deaminase deficiency-related severe combined immunodeficiency (ADA-SCID), Duchenne (DMD) and Becker muscular dystrophy (BMD), Parkinson disease (PD) or juvenile-onset type 1 diabetes mellitus (JDM).

## Patentansprüche

1. Die Verwendung eines pseudotypisierten lentiviralen Vektorpartikels in der gezielten *in vitro* Transduktion von undifferenzierten pluripotenten humanen embryonalen Stammzellen (hESC) und von induzierten pluripotenten Stammzellen (iPSC), wobei jener Vektor ein Morbillivirus Fusionsprotein (F) und ein mutiertes Hämagglutininprotein (H) des Masernvirus (MeV) oder des Edmonton Stammes des Masernvirus (MeV_{Edm}) umfasst,
worin die cytoplasmatischen Teile des F und des H Proteins gekürzt sind und das gekürzte F Protein FcΔ24 ist (24 Aminosäuren entfernt vom C-Terminus) oder FcΔ30 ist (30 Aminosäuren entfernt vom C-Terminus) und das mutierte und gekürzte H Protein ausgewählt ist aus der Gruppe bestehend aus HcΔ14 (Aminosäuren 2-15 entfernt), HcΔ15 (Aminosäuren 2-16 entfernt), HcΔ16 (Aminosäuren 2-17 entfernt), HcΔ17 (Aminosäuren 2-18 entfernt), HcΔ18 (Aminosäuren 2-19 entfernt), HcΔ19 (Aminosäuren 2-20 entfernt), HcΔ20 (Aminosäuren 2-21 entfernt), HcΔ21+A (Aminosäuren 2-22 ersetzt durch ein Alanin) und HcΔ24+4A (Aminosäuren 2-25 ersetzt durch vier Alanine), bevorzugt HcΔ18, HcΔ19 und HcΔ24+4A,
worin die Aminosäuren, die notwendig für die Rezeptorerkennung im H Protein sind, so mutiert sind, dass es nicht mit CD46, SLAM und/oder Nectin-4 interagiert und worin das H Protein ferner einen Einzelketten-Antikörper gerichtet gegen CD30, EpCAM (CD326), CD9, Thy-1 (CD90), SSEA-3, SSEA-4, TRA-1-60 oder TRA-1-81 an seiner Ektodomäne hat.

2. Die Verwendung nach Anspruch 1, worin die Mutation im H Protein ist:
- mindestens eine Punktmutation mit einer anderen Aminosäure an den Resten ausgewählt aus der Gruppe bestehend aus V451, Y529, Y481, F431, V451, Y452, A527, P486, I487, A428, L464, R533, G546, S548, und F549 mit Alanin, Leucin, Serin, Methionin oder Glutamin und/oder
- einer Punktmutation aller fünf aufeinanderfolgender Reste 473 bis 477 mit Alanin und/oder
- mindestens eine Punktmutation mit einer anderen Aminosäure an den Resten ausgewählt aus der Gruppe bestehend aus I194, D530, Y553, T531, P554, F552, D505, und D507 und/oder
- mindestens eine Punktmutation mit einer anderen Aminosäure an den Resten ausgewählt aus der Gruppe bestehend aus Y543 und P497.

3. Die Verwendung nach einem der Ansprüche 1 bis 2, worin das scFv gegen CD30 gerichtet ist, die eine optimierte Aminosäuresequenz wie in Fig. 5 gezeigt hat.

4. Die Verwendung nach einem der Ansprüche 1 bis 3, worin der pseudotypisierte lentivirale Vektorpartikel von einem Lentivirus ausgewählt aus der Gruppe bestehend aus HIV-1, HIV-2, SIV_{mac}, SIV_{pbj}, SIV_{agm}, FIV und EIAV, bevorzugt HIV-1 abgeleitet ist.

5. Die Verwendung nach Anspruch 4, worin der pseudotypisierte lentivirale Vektorpartikel nicht die genetische Information von *gag, env* und/oder *pol* Genen umfasst und/oder von anderen nicht essentiellen Genen, die ausgewählt sind aus der Gruppe bestehend aus *tat, vif, vpr, vpu,* und *nef.*

6. Die Verwendung nach Anspruch 5, worin jene Gene *in trans* durch eine Verpackungszelllinie bereitgestellt werden, die ausgewählt ist aus der Gruppe bestehend aus humanen embryonalen Nieren 293 Zellen, die SV40 großes T-Antigen enthalten (HEK-293T oder 293T), menschliche Sarkomazelllinie HAT-1080 (CCL-121), Lymphoblasten-ähnliche Zelllinie Raji (CCL-86), Glioblastoma-Astrocytoma Epithelähnliche Zelllinie U87-MG (HTB-14) und T-Lymphom Zelllinie HuT78 (TIB-161), bevorzugter HEK-293T.

7. Die Verwendung nach einem der Ansprüche 1 bis 6, worin die Menge an gekürztem F Protein, die in jenem Vektorpartikel vorliegt, höher ist als die Menge an mutiertem und gekürztem H Protein, die in jenem Vektorpartikel vorliegt, bevorzugt 10-100%, 250-500%, 500-750%, 750-1000% oder mehr als 1000% höher und, bevorzugt, 700% höher ist als die Menge an mutiertem und gekürztem H Protein.

8. Die Verwendung nach einem der Ansprüche 1 bis 7, ferner umfassend einen psipositiven RNA Expressionsvektor, worin der psi-positive RNA Expressionsvektor mindestens ein selektierbares Marker-Gen umfasst.

9. Die Verwendung nach Anspruch 8, worin das selektierbare Marker-Gen ausgewählt ist aus der Gruppe bestehend aus ein Gen, das für GFP kodiert, ein Gen, das für eGFP kodiert, ein Gen, das für ein Apoptose-induzierendes Protein kodiert, ein Gen, das für ein cytotoxisches Protein kodiert, TNF-α Gen, p53 Gen, eine interferierende RNA, ein Interferon Gen, das Herpes Virus Thymidin Kinase Gen, ein Gen, das für ein immunstimulierendes Protein kodiert und ein Gen, das für ein therapeutisches Protein kodiert.

10. Die Verwendung nach einem der Ansprüche 1-9, worin die iPSCs ausgewählt sind aus der Gruppe bestehend aus hFFBiPS SB4 und HFFBiPS SB5 oder von Patienten generiert sind, die Adenosine-deaminase deficiency-related severe combined immunodeficiency (ADA-SCID), Duchenne (DMD) und Becker muscular dystophy (BMD), Parkinson (PD) oder juvenile-onset type-1 diabetes mellitus (JDM) haben.

## Revendications

1. Utilisation d'une particule vecteur lentivirale pseudotypée dans la traduction ciblée *in vitro* des cellules souche embryonnaires humaines pluripotentes non différenciées (hESC) et cellules souches pluripotents induites (iPSC), le vecteur comprenant une protéine de fusion (F) de morbillivirus et une protéine hémagglutinine (H) du virus de la rougeole (MeV) de la souche Edmonton du virus de la rougeole (MeVEdm),
dans laquelle les portions cytoplasmiques de la protéine F et H sont tronquées et la protéine F tronqué est FcΔ24 (24 acides aminés retranchés de l'extrémité C-terminale) ou FcΔ30 (30 acides aminées retranchés de l'extrémité C-terminale) et la protéine H mutée et tronquée et choisie parmi le groupe consisté d'HcΔ14 (acides aminées 2-15 retranchés), HcΔ15 (acides aminés 2-16 retranchés), HcΔ16 (acides aminés 2-17 retranchés), HcΔ17 (acides aminés 2-18 retranchés), HcΔ18 (acides aminés 2-19 retranchés), HcΔ19 (acides aminés 2-20 retranchés), HcΔ20 (acides aminés 2-21 retranchés), HcΔ21+A (acides aminés 2-22 remplacés par une alanine) et HcΔ24+4A (acides aminés 2-25 remplacés par quatre alanines), de préférence HcΔ18, HcΔ19 et HcΔ24+4A,
dans laquelle les acides aminés étant nécessaires pour la reconnaissance du récepteur dans la protéine H sont mutés de telle sorte qu'elle n'interagit pas avec CD46, SLAM et/ou la nectine-4 et dans laquelle la protéine H présente en outre un anticorps à chaîne simple ciblé à CD30, EpCAM (CD326), CD9, Thy-1 (CD90), SSEA-3, SSEA-4, TRA-1-60 ou TRA-1-81 au niveau de son ectodomaine.

2. Utilisation selon la revendication 1, dans laquelle la mutation dans la protéine H est:
- au moins une mutation ponctuelle avec un autre acide aminé au niveau des résidus choisis parmi le groupe constitué de V451, Y529, Y481, F431, V451, Y452, A527, P486, I487, A428, L464, R533, G546, S548 et F549 avec l'alanine, la leucine, la serine, la méthionine ou la glutamine; et/ou
- une mutation ponctuelle de chacun des cinq résidus consécutives 473 à 477 avec l'alanine; et/ou
- au moins une mutation ponctuelle avec un autre acide aminé au niveau des résidus choisis parmi le groupe constitué d'I194, D530, Y553, T531, P554, F552, D505 et D507; et/ou
- au moins une mutation ponctuelle avec un autre acide aminé au niveau des résidus choisis parmi le groupe constitué d'Y543 et P497.

3. Utilisation selon l'une quelconque des revendications 1 à 2, dans laquelle le scFv est ciblé à CD30 ayant la séquence d'acides aminés optimisé, comme indiqué sur la figure 5.

4. Utilisation selon l'une quelconque des revendications 1 à 3, dans laquelle la particule vecteur lentivirale pseudotypée est dérivée d'un lentivirus choisi parmi le groupe constitué de VIH-1, VIH-2, VIS_{mac}, VIS_{pbj}, VIS_{agm}, VIF et VAIE, de préférence VIH-1.

5. Utilisation selon la revendication 4, dans laquelle la particule vecteur lentivirale pseudotypée ne contient pas d'information génétique des gènes *gag, env* et/ou *pol* et/ou d'autres gènes non essentiels qui sont choisis parmi le groupe constitué de *tat, vif, vpr, vpu* et *nef.*

6. Utilisation selon la revendication 5, dans laquelle lesdites gènes sont fournis dans la manière trans au moyen d'une lignée cellulaire, qui est choisie parmi le groupe constitué de cellules embryonnaires rénale 293, qui contient l'antigène T large de SV40 (HEK-293T ou 293T), la lignée cellulaire de sarcome humaine HT-1080 (CCL-121), la lignée cellulaire du type lymphoblastique Raji (CCL-86), la lignée cellulaire du type épithélial glioblastome-astrocytome U87-MG (HTB-14) et la lignée cellulaire de lymphome T HuT78 (TIB-161), plus préférablement HEK-293T.

7. Utilisation selon l'une quelconque des revendications 1 à 6, dans laquelle la quantité de la protéine F tronquée, qui est présente dans ladite particule vecteur, est plus élevée que la quantité de la protéine H mutée et tronquée, qui est présente dans ladite particule vecteur, de préférence de 10-100%, 100-250%, 250-500%, 500-750%, 750-1000% ou plus que 1000% plus élevée et, de préférence de 700% plus élevée que la quantité de la protéine H mutée et tronquée.

8. Utilisation selon l'une quelconque des revendications 1 à 7, comprenant en outre un vecteur d'expression d'ARN psi-positive, dans lequel le vecteur d'expression d'ARN psi-positive comprend au moins un gène marquer sélectionnable.

9. Utilisation selon la revendication 8, dans laquelle le gène marquer sélectionnable est choisi parmi le groupe constitué d'un gène codant la GFP, d'un gène codant la eGFP, d'un gène codant une protéine, qui induit l'apoptose, d'un gène codant une protéine cytotoxique, d'un gène TNF-α, d'un gène p53, d'un ARN interférant, d'un gène d'interféron, du gène de thymidine kinase de virus de l'herpès, d'un gène codant une protéine immunostimulante et d'un gène codant une protéine thérapeutique.

10. Utilisation selon l'une quelconque des revendications 1 à 9, dans laquelle les iPSCs sont choisies parmi le groupe constitué d' hFFBiPS SB4 et HFFBiPS SB5 ou sont générées à partir de patients présentant le déficit immunitaire combiné sévère par déficit en adénosine désaminase (ACA-SCID), la myopathie de Duchenne (DMD) et Becker (BMD), la maladie de Parkinson (PD) ou le diabète de type 1 à début juvénile.
